(19) Europäisches Patentamt
European Patent Office
Office européen des brevets

(11) EP 2 640 417 B1

# (12) EUROPEAN PATENT SPECIFICATION

(45) Date of publication and mention of the grant of the patent:
**01.04.2020 Bulletin 2020/14**

(21) Application number: **11841437.4**

(22) Date of filing: **07.11.2011**

(51) Int Cl.:
*A61K 39/002* (2006.01) *A61K 48/00* (2006.01)
*A61K 38/17* (2006.01) *A61P 31/12* (2006.01)

(86) International application number:
**PCT/US2011/059501**

(87) International publication number:
**WO 2012/067866 (24.05.2012 Gazette 2012/21)**

## (54) MULTIVALENT VACCINE FOR FILARIASIS

MULTIVALENTER IMPFSTOFF GEGEN FILARIOSE

VACCIN MULTIVALENT POUR LA FILARIOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2011 US 201161449954 P**
**10.08.2011 US 201161522079 P**
**15.11.2010 US 413681 P**

(43) Date of publication of application:
**25.09.2013 Bulletin 2013/39**

(73) Proprietor: **The Board of Trustees of the University of Illinois**
**Urbana, IL 61801 (US)**

(72) Inventor: **KALYANASUNDARAM, Ramaswamy**
**Rockford, IL 61107 (US)**

(74) Representative: **FRKelly**
**27 Clyde Road**
**Dublin D04 F838 (IE)**

(56) References cited:

- **ABHILASH SAMYKUTTY ET AL: "Multivalent Vaccine for Lymphatic Filariasis", PROCEDIA IN VACCINOLOGY, vol. 3, 1 January 2010 (2010-01-01), pages 12-18, XP055112909, ISSN: 1877-282X, DOI: 10.1016/j.provac.2010.11.003**
- **DASH YASHODHARA ET AL: "Granuloma Formation around Filarial Larvae Triggered by Host Responses to an Excretory/Secretory Antigen", INFECTION AND IMMUNITY, vol. 79, no. 2, 10 April 2010 (2010-04-10) , pages 838-845, XP55112913,**
- **BENJAMIN L. MAKEPEACE ET AL: "Immunisation with a Multivalent, Subunit Vaccine Reduces Patent Infection in a Natural Bovine Model of Onchocerciasis during Intense Field Exposure", PLOS NEGLECTED TROPICAL DISEASES, vol. 3, no. 11, 10 November 2009 (2009-11-10), page e544, XP055112924, DOI: 10.1371/journal.pntd.0000544**
- **SETTY BALAKRISHNAN ANAND ET AL.: 'Comparison of immunogenicity, protective efficacy of single and cocktail DNA vaccine of Brugia malayi abundant larval transcript (ALT-2) and thioredoxin peroxidase (TPX) in mice' ACTA TROPICA vol. 107, no. 2, 2008, pages 106 - 112, XP023176533**
- **MUNIRATHINAM GNANASEKAR ET AL.: 'Identification and cloning of a novel tetraspanin (TSP) homologue from Brugia malayi' DNA SEQUENCE vol. 19, no. 2, 2008, pages 151 - 156, XP055105849**
- **WILLIAM F. GREGORY ET AL.: 'The abundant larval transcript-1 and -2 genes of Brugia malayi encode stage-specific candidate vaccine antigens for filariasis' INFECTION AND IMMUNITY vol. 68, no. 7, 2000, pages 4174 - 4179, XP055105506**
- **SIMON A. BABAYAN ET AL.: 'Vaccination against filarial nematodes with irradiated larvae provides long-term protection against the third larval stage but not against subsequent life cycle stages' INTERNATIONAL JOURNAL FOR PARASITOLOGY vol. 36, no. 8, 2006, pages 903 - 914, XP028070298**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 640 417 B1

## Description

### Introduction

This patent application claims the benefit of priority from U.S. Provisional Application Serial Number 61/413,681, filed November 15, 2010; U.S. Provisional Application Serial Number 61/449,954, filed March 7, 2011 and from U.S. Provisional Application Serial Number 61/522,079, filed August 10, 2011.

This invention was made with government support under contract number 5R01AI064745-04 awarded by the National Institutes of Health. The government has certain rights in the invention.

### Background of the Invention

Lymphatic filariasis caused by the filarial nematodes *Wuchereria bancrofti, Brugia malayi,* and *Brugia timori,* affects more than 120 million people worldwide (WHO (1992) World Health Organ. Tech. Rep. Ser. 821:1-71). Mass drug administration program by the World Health Organization, is significantly reducing the incidence rate of lymphatic filariasis in many parts of the world (Hotez (2009) Clin. Pharmacol. Ther. 85(6):659-64). Nevertheless, lack of effectiveness to the mass drug administration has been reported from several endemic regions mainly due to noncompliance (Babu & (2008) Trans. R. Soc. Trop. Med. Hyg. 102(12):1207-13; El-Setouhy, et al. (2007) Am. J. Trop. Med. Hyg. 77(6):1069-73). In addition, drug resistance has been reported to at least one of the drugs in the mass drug combination (Horton (2009) Ann. Trop. Med. Parasitol. 103(1):S33-40; Schwab, et al. (2007) Parasitology 134(Pt 7):1025-40). Since yearly administration of the mass drugs is required for effective control, there is an alarming concern for selecting drug resistant parasites. Therefore, there is an immediate need for a multipronged approach in controlling this mosquito borne infection.

Vaccination is one strategy for controlling this infection and several subunit candidate vaccine antigens have been tested in laboratory animals with variable results (Bottazzi, et al. (2006) Expert Rev. Vaccines 5(2):189-98; Chenthamarakshan, et al. (1995) Parasite Immunol. 17(6):277-85; Dissanayake, et al. (1995) Am. J. Trop. Med. Hyg. 53(3):289-94; Li, et al. (1993) J. Immunol. 150(5):1881-5; Maizels, et al. (2001) Int. J. Parasitol. 31(9):889-98; Thirugnanam, et al. (2007) Exp. Parasitol. 116(4):483-91; Veerapathran, et al. (2009) PLoS Negl. Trop. Dis. 3(6):e457). Lymphatic filariasis is a multicellular organism with complex life cycle and produce large array of host modulatory molecules. Thus, fighting against this infection with a single antigen vaccine can be difficult. By screening a phage display cDNA expression library of the *B. malayi* parasite with sera from immune individuals, several potential vaccine candidates were identified (Gnanasekar, et al. (2004) Infect. Immun. 72(8):4707-15). However, a varying degree of protection was achieved with each of the candidate vaccine antigens when given as a DNA, protein or prime boost vaccine (Veerapathran, et al. (2009) *supra*).

Anand et al., 2008 discloses a cocktail vaccine based on plasmids encoding larval (L3) stage-specific Brugia malayi abundant larval transcript (BmALT-2) and antioxidant detoxification enzyme B. malayi thioredoxin peroxidase (BmTPX) to induce antibodies, protective efficacy and cell-mediated immune response in mice. The mice administered with cocktail vaccine induced up to 78% of cytotoxicity against B. malayi mf. This cytotoxicity was high compared to 34% induced by the pVAX-ALT2 or 37% by pVAX-TPX.

Gnanasekar et al., 2008 discloses a tetraspanin (TSP)-like molecule in the lymphatic filarial parasites. The full length gene cloned from B. malayi showed significant similarity to Caenorhabditis elegans TSP and human TSP and hence the gene was named B. malayi TSP (BmTSP).

Samykutty et al., 2010 discloses a multivalent DNA-based vaccine comprising BmALT-2 and BmHSP antigens of lymphatic filariasis. Nearly 90% protection can be achieved using the multivalent formulation in a DNA prime protein boost approach.

### Summary of the Invention

The present invention features a multivalent vaccine composed of a fusion protein comprising three or more covalently attached isolated antigens from one or more filarial nematodes wherein the antigens comprise Abundant Larval Transcript (ALT-2), Tetraspanin, and Small heat shock protein (HSP) 12.6, or fragments thereof, wherein the fragment is selected from the group of SEQ ID NO:39, SEQ ID NO:63, and SEQ ID NO:64. In certain embodiments, the filarial nematodes are selected from the group of *Brugia malayi, Wuchereria Bancroft, Brugia timori, Onchocerca volvulus* and *Loa loa.* In one embodiment, the antigens are protein-based, DNA-based, or a combination thereof. The antigens are covalently attached. The antigens include Abundant Larval Transcript (ALT-2), Tetraspanin, Small heat shock protein (HSP) 12.6, or fragments thereof including, *e.g.,* SEQ ID NO:39, SEQ ID NO:63, SEQ ID NO:64, SEQ ID NO:65, SEQ ID NO:66, SEQ ID NO:67, SEQ ID NO:68, and SEQ ID NO:69. Recombinant vectors containing one or more antigens of one or more filarial nematodes are also disclosed as are recombinant host cells containing the same.

Also disclosed is a method for immunizing an animal against filariasis by administering to an animal in need thereof a multivalent vaccine of the present invention.

**Brief Description of the Drawings**

Figure 1 shows the titer of anti-BmHSP and anti-BmALT-2 IgG antibodies in the sera of vaccinated mice. 6-week-old balb/c mice were immunized using a prime boost approach with a monovalent vaccine (*Bmhsp* prime and rBmHSP boost or *Bmalt-2* prime and rBmALT-2 boost) and multivalent vaccine (*Bmhsp*/*Bmalt-2* prime and rBmHSP and rBmALT-2 boost). Titer of IgG antibodies were measured in the sera using an indirect ELISA. The data presented is the antibody titer 2 weeks after the last booster. Results show that both bivalent and multivalent vaccines induce significant IgG antibodies against each of the component antigens. The findings also show that the antigens in the monovalent and multivalent formulations act synergistically in boosting the immune responses. N=5. Statistically significant ** $p < 0.001$, * $p < 0.05$. Values represented are mean ± SD.

Figure 2 shows the number of IL-4 (Figure 2A) and IFN-γ (Figure 2B) secreting cells in the spleen of mice vaccinated with monovalent (BmHSP or BmALT-2) or multivalent vaccine. An ELISPOT assay was performed after stimulating the cells with rBmHSP or rBmALT (1 μg/ml). Single cell preparations of spleen cells were stimulated with respective antigens for 48 hours and spot forming cells were counted. Results show that both monovalent and multivalent vaccine promoted IL-4 secreting cells. Multivalent vaccination induced the higher number of IL-4 producing cells than controls. IFN-γ producing cells were comparatively low. These findings further confirm that BmHSP and BmALT-2 synergistically boost the immune responses in vaccinated animals following a multivalent vaccination. N=5. Results are expressed as mean number of spot forming units per 3 x $10^6$ cells ± SD.

Figure 3 shows the degree of protection conferred by a multivalent vaccine in a mouse model. Balb/c strain of mice were immunized with HAT (HSP/ALT-2/TSP) hybrid DNA, with recombinant HAT protein or a combination of both using a prime boost approach. HAT hybrid DNA was used for priming. Two weeks following the priming, mice were boosted with HAT hybrid protein. Another group of mice were immunized with HAT hybrid DNA or with HAT hybrid protein. Control groups of mice received only blank vector or alum adjuvant. Two weeks after the last immunization, mice were challenged with 20 infective larvae of *Brugia malayi* by placing them in a micropore chamber in the peritoneal cavity of the immunized mice. After 48 hours, larval death was measured to determine the success of vaccination.

Figure 4 shows a western blot of recombinant BmTSP (lane 1) and two different fractions of isolated, recombinant *O. volvulus* TSP (lanes 2 and 3) probed with sera from cHAT-vaccinated mice.

**Detailed Description of the Invention**

A multivalent vaccine for filariasis has now been developed. Combinations of antigens, Abundant Larval Transcript (ALT-2), Tetraspanin (TSP), Small heat shock protein (HSP) 12.6, were prepared into one hybrid DNA antigen or one fusion protein antigen. When tested in experimental animals (i.e., mouse, jirds, mastomys), the combination of hybrid DNA plus hybrid protein vaccination gave 100% protection. Hybrid protein alone vaccine gave >80% protection. Accordingly, the present invention features multivalent protein-based and DNA-based vaccines composed of filarial nematode antigens as defined in the claims or nucleic acids encoding the same and use of the vaccines to prevent or control filariasis in humans and animals.

For the purposes of the present invention, a multivalent or polyvalent vaccine refers to a vaccine prepared from three or more covalently attached antigens as defined in the claims. According to some embodiments, the antigen is a nucleic acid molecule, which is referred to herein as a "DNA-based" antigen. According to other embodiments, the antigen is a protein or polypeptide, which is referred to herein as "protein-based" antigen. A multivalent vaccine of the invention can be composed of three antigens as defined in the claims or their fragments in various permutation combinations. In some embodiments, the multivalent vaccine is composed of solely of protein antigens. In other embodiments, the multivalent vaccine is composed solely of DNA-based antigens. The multivalent vaccine may also be composed of a mixture of protein- and DNA-based antigens.

Antigens disclosed herein can be provided or expressed from a single nucleic acid molecule containing, *e.g.,* internal ribosome entry sites between the antigens. Moreover, the antigens of the multivalent vaccine of this invention can be covalently attached to form a hybrid or chimeric molecule, wherein the antigens are immediately adjacent to one another *(e.g.,* an in-frame fusion with or without a short spacer). Alternatively, the antigens can be provided as a mixture of individual antigens. Moreover, it is contemplated that the instant vaccine can be composed of a hybrid molecule.

In one embodiment, the antigens of the multivalent vaccine as defined in the claims are different proteins from one species of filarial nematode. As an example of this embodiment, the multivalent vaccine is composed of ALT-2, TSP and HSP antigens isolated from one or more strains of *B. malayi.*. In yet a further embodiment, the multivalent vaccine as defined in the claims of a combination of different antigens from different species of filarial nematodes.

For preparing multivalent DNA vaccines or multivalent recombinant DNA vaccines, the DNA sequence of the gene of interest (also used interchangeably as DNA molecule) need not contain the full length of DNA encoding the corresponding protein. Likewise, when preparing multivalent protein-based vaccines or multivalent recombinant protein vaccines, the protein sequence need not contain the full length protein. In most cases, a fragment of the protein or gene which encodes an epitope region is sufficient for immunization. The DNA/protein sequence of an epitope region can be found by sequencing the corresponding part of the gene from various strains or species and comparing them. The major antigenic determinants are likely to be those showing the greatest heterology. Also, these regions are likely to lie accessibly in the conformational structure of the proteins. One or more such fragments of proteins or genes encoding the antigenic determinants can be prepared by chemical synthesis or by recombinant DNA technology. These fragments of proteins or genes, if desired, can be linked together or linked to other proteins or DNA molecules, respectively.

As described herein, the ALT-2, TSP, and HSP antigens were identified as providing protection against infection by filaria larvae. Accordingly, in particular embodiments, the instant multivalent vaccine includes a fusion protein composed of ALT-2, TSP, and HSP protein antigens or fragments as defined in the claims or a nucleic acid encoding said fusion protein as also defined in the claims. Protein and nucleic acid sequences for these antigens are available in the art under the GENBANK accession numbers listed in Table 1.

TABLE 1

| Antigen | Source | Protein | SEQ ID NO: | Nucleic Acid | SEQ ID NO: |
|---|---|---|---|---|---|
| ALT-2 | *B. malayi* | P90708 XP_001896203 | 37 39 | BMU84723 XM_001896168 | 38 40 |
| | *W. bancrofti* | AAC35355 | 41 | AF084553 | 42 |
| | *L. loa* | XP_003151340 | 43 | XM_ 003151292 | 44 |
| TSP | *B. malayi* | ABN55911 | 45 | EF397425 | 46 |
| | *L. loa* | XP_003136177 | 47 | XM_003136129 | 48 |
| HSP | *B. malayi* | AAU04396 | 49 | AY692227 | 50 |
| | *O. volvulus* | CAA48633 | 51 | X68669 | 52 |
| | *L. loa* | XP 003139338 | 53 | XM_ 003139290 | 54 |
| VAL-1 | *B. malayi* | AAB97283 | 55 | AF042088 | 56 |
| | *W. bancrofti* | AAD16985 | 57 | AF109794 | 58 |
| | *O. volvulus* | AAB69625 | 59 | AF020586 | 60 |
| | *L. loa* | XP_003146897 | 61 | XM_003146849 | 62 |

In addition, the nucleotide sequence encoding *O. volvulus* TSP can be found under GENBANK Accession No. JN861043. The protein antigens and nucleic acid molecules of the invention can be used as full length molecules or less than full length molecules. In this respect, the present invention further includes the use of fragments of the above-referenced protein antigens and nucleic acid molecules and they are also defined in the claims. Exemplary nucleic acid fragments may include 20, 30, 40, 50, 60, 70, 80, 90, 100, 150, or 200 amino acid residue portions of full-length protein antigens (*e.g.,* those listed in Table 1) or 60, 90, 120, 150, 180, 210, 240, 270, 300, 350, or 600 nucleotide portion of full-length nucleic acid molecules (*e.g.,* those listed in Table 1). Exemplary protein fragments include the extracellular loop (ECL) domain of TSP (see, *e.g.,* the ECL domain of *W.* bancrofti TSP of SEQ ID NO:63) and N-terminal deletion of HSP 12.6 (cHSP; see, *e.g.,* the *W.* bancrofti HSP fragment of SEQ ID NO:64), A vaccine can further include other known antigens from *W. bancrofti, B. malayi, O. volvulus, L. loa* and *B. timori.* Examples of other suitable antigens include, but are not limited to, glutathione peroxidase (see Cookson, et al. (1992) Proc. Natl. Acad. Sci. USA 89:5837-5841; Maizels, et al. (1983) Parasitology 87:249-263; Maizels, et al. (1983) Clin, Exp. Immunol. 51:269-277); recombinant antigen (*BmR1;* see Noordin, et al. (2004) Filaria J. 3:10); class II aminoacyl-tRNA synthetase (see Kron, et al. (1995) FEBS Lett. 374:122-4); heat shock cognate 70 (hsc70) protein (see Selkirk, et al. (1989) J. Immunol. 143:299-308); and paramyosin (see Li, et al. (1991) Mol. Biochem. Parasitol. 49:315-23) . The antigen can be obtained from a filarial nematode selected from the group of *W. bancrofti, B. malayi, O. volvulus, L.* loa and *B. timori.*

As disclosed herein, the antigens of the multivalent vaccine are isolated from a filarial nematode. In this respect, an isolated nucleic acid molecule or protein is a nucleic acid molecule or protein that has been removed from its natural milieu (*i.e*., that has been subjected to human manipulation). As such, "isolated" does not reflect the extent to which the nucleic acid molecule or protein has been purified (*e.g.*, purified to greater than 95% homogeneity). An isolated and optionally purified nucleic acid molecule or protein as disclosed herein can be obtained from its natural source or produced using recombinant DNA technology (*e.g.*, polymerase chain reaction (PCR) amplification or cloning) or chemical synthesis. Isolated nucleic acid molecules and proteins can also include, for example, natural allelic variants or isomers that induce an immune response in the host.

One embodiment of the present invention includes a recombinant vector, which includes at least one isolated nucleic acid molecule of the present invention, inserted into a vector capable of delivering the nucleic acid molecule into a host cell. Such a vector contains heterologous nucleic acid sequences, that are nucleic acid sequences that are not naturally found adjacent to nucleic acid molecules of the present invention and that preferably are derived from a species other than the species from which the nucleic acid molecule(s) are derived. The vector can be either prokaryotic or eukaryotic, and typically is a virus or a plasmid. Recombinant vectors can be used in the cloning, sequencing, and/or otherwise manipulating the nucleic acid molecules of the present invention.

The present disclosure also includes an expression vector, which includes a nucleic acid molecule of the present invention in a recombinant vector that is capable of expressing the nucleic acid molecule when transformed into a host cell. Preferably, the expression vector is also capable of replicating within the host cell. Expression vectors can be either prokaryotic or eukaryotic, and are typically viruses or plasmids. Expression vectors as disclosed herein include any vectors that function (*i.e.*, direct gene expression) in recombinant cells of the present invention, including in bacterial, fungal, parasite, insect, other animal, and plant cells. For example, expression vectors can direct gene expression in bacterial, yeast, helminth or other parasite, insect and mammalian cells.

Alternatively they may contain regulatory sequences such as transcription control sequences, translation control sequences, origins of replication, and other regulatory sequences that are compatible with the recombinant cell and that control the expression of nucleic acid molecules of the present invention. They may also include transcription control sequences. Transcription control sequences are sequences which control the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the recombinant cells of the present invention. A variety of such transcription control sequences are known to those skilled in the art. Preferred transcription control sequences include those which function in bacterial, yeast, helminth or other endoparasite, or insect and mammalian cells, such as, but not limited to, tac, lac, trp, trc, oxy-pro, omp/lpp, rrnB, bacteriophage lambda (such as lambda $p_L$ and lambda $p_R$ and fusions that include such promoters), bacteriophage T7, T7lac, bacteriophage T3, bacteriophage SP6, bacteriophage SP01, metallothionein, alpha-mating factor, *Pichia* alcohol oxidase, alphavirus subgenomic promoter, antibiotic resistance gene, baculovirus, Heliothis zea insect virus, vaccinia virus, herpesvirus, raccoon poxvirus, other poxvirus, adenovirus, cytomegalovirus (such as immediate early promoter), simian virus 40, retrovirus, actin, retroviral long terminal repeat, Rous sarcoma virus, heat shock, phosphate and nitrate transcription control sequences as well as other sequences capable of controlling gene expression in prokaryotic or eukaryotic cells. Additional suitable transcription control sequences include tissue-specific promoters and enhancers as well as lymphokine-inducible promoters (*e.g.*, promoters inducible by interferons or interleukins). Transcription control sequences of the present invention can also include naturally occurring transcription control sequences naturally associated with parasitic helminths, such as B. *malayi* transcription control sequences.

Recombinant molecules of the present invention may also contain (a) secretory signals (*i.e.*, signal segment nucleic acid sequences) to enable an expressed protein of the present invention to be secreted from the cell that produces the protein and/or (b) fusion sequences which lead to the expression of nucleic acid molecules of the present invention as fusion proteins. Examples of suitable signal segments include any signal segment capable of directing the secretion of a protein of the present invention. Preferred signal segments include, but are not limited to, tissue plasminogen activator (t-PA), interferon, interleukin, growth hormone, histocompatibility and viral envelope glycoprotein signal segments. In addition, a nucleic acid molecule of the present invention can be joined to a fusion segment that directs the encoded protein to the proteosome, such as a ubiquitin fusion segment. Eukaryotic recombinant molecules may also include intervening and/or untranslated sequences surrounding and/or within the nucleic acid sequences of nucleic acid molecules of the present invention.

Another embodiment of the present invention includes a recombinant host cell harboring one or more recombinant molecules of the present invention. Transformation of a nucleic acid molecule into a cell can be accomplished by any method by which a nucleic acid molecule can be inserted into the cell. Transformation techniques include, but are not limited to, transfection, electroporation, microinjection, lipofection, adsorption, and protoplast fusion. A recombinant cell may remain unicellular or may grow into a tissue, organ or a multicellular organism. Transformed nucleic acid molecules of the present invention can remain extrachromosomal or can integrate into one or more sites within a chromosome of the transformed (*i.e.*, recombinant) cell in such a manner that their ability to be expressed is retained.

Suitable host cells to transform include any cell that can be transformed with a nucleic acid molecule of the present invention. Host cells can be either untransformed cells or cells that are already transformed with at least one nucleic acid molecule (*e.g.,* nucleic acid molecules encoding one or more fusion proteins of the present invention and/or other proteins useful in the production of multivalent vaccines). Host cells of the present invention either can be endogenously (*i.e.*, naturally) capable of producing fusion proteins of the present invention or can be capable of producing such proteins after being transformed with at least one nucleic acid molecule of the present invention. Host cells of the present invention can be any cell capable of producing at least one protein of the present invention, and include bacterial, fungal (including yeast), parasite (including helminth, protozoa and ectoparasite), other insect, other animal and plant cells. Preferred host cells include bacterial, mycobacterial, yeast, helminth, insect and mammalian cells. More preferred host cells include *Salmonella, Escherichia, Bacillus, Listeria, Saccharomyces, Spodoptera, Mycobacteria, Trichoplusia,* BHK (baby hamster kidney) cells, MDCK cells (Madin-Darby canine kidney cell line), CRFK cells (Crandell feline kidney cell line), CV-1 cells (African monkey kidney cell line used, for example, to culture raccoon poxvirus), COS (*e.g.*, COS-7) cells, and Vero cells. Particularly preferred host cells are *Escherichia coli,* including *E. coli* K-12 derivatives; *Salmonella typhi; Salmonella typhimurium; Spodoptera frugiperda; Trichoplusia ni;* BHK cells; MDCK cells; CRFK cells; CV-1 cells; COS cells; Vero cells; and non-tumorigenic mouse myoblast G8 cells (*e.g.*, ATCC CRL 1246). Additional appropriate mammalian cell hosts include other kidney cell lines, other fibroblast cell lines (*e.g.*, human, murine or chicken embryo fibroblast cell lines), myeloma cell lines, Chinese hamster ovary cells, mouse NIH/3T3 cells, LMTK$^{31}$ cells and/or HeLa cells. In one embodiment, the proteins may be expressed as heterologous proteins in myeloma cell lines employing immunoglobulin promoters.

A recombinant cell is preferably produced by transforming a host cell with one or more recombinant molecules, each comprising one or more nucleic acid molecules of the present invention and one or more transcription control sequences, examples of which are disclosed herein.

Recombinant DNA technologies can be used to improve expression of transformed nucleic acid molecules by manipulating, for example, the number of copies of the nucleic acid molecules within a host cell, the efficiency with which those nucleic acid molecules are transcribed, the efficiency with which the resultant transcripts are translated, and the efficiency of post-translational modifications. Recombinant techniques useful for increasing the expression of nucleic acid molecules of the present invention include, but are not limited to, operatively linking nucleic acid molecules to high-copy number plasmids, integration of the nucleic acid molecules into one or more host cell chromosomes, addition of vector stability sequences to plasmids, substitutions or modifications of transcription control signals (*e.g.,* promoters, operators, enhancers), substitutions or modifications of translational control signals (*e.g.*, ribosome binding sites, Shine-Dalgarno sequences), modification of nucleic acid molecules of the present invention to correspond to the codon usage of the host cell, deletion of sequences that destabilize transcripts, and use of control signals that temporally separate recombinant cell growth from recombinant enzyme production during fermentation. The activity of an expressed recombinant protein of the present invention may be improved by fragmenting, modifying, or derivatizing nucleic acid molecules encoding such a protein. Moreover, while non-codon-optimized sequences may be used to express fusion proteins in host cells such as *E. coli* (see Table 1), in embodiments pertaining to DNA vaccines, the nucleic acid molecule may be codon-optimized to facilitate expression in mammalian cells. In this respect, codon-optimized sequences for BmALT-2, N-terminal deleted HSP 12.6 (cHSP) of *W. bancrofti,* and ECL domain of *W. bancrofti* Tetraspanin are set forth in SEQ ID NO:67, SEQ ID NO:68, and SEQ ID NO:69, respectively.

Isolated protein-based antigens as disclosed herein or the fusion protein of the present invention can be produced in a variety of ways, including production and recovery of natural proteins, production and recovery of recombinant proteins, and chemical synthesis of the proteins. In one embodiment, fusion protein of the present invention is produced by culturing a cell capable of expressing the fusion protein under conditions effective to produce the protein, and recovering the fusion protein. A preferred cell to culture is a recombinant cell of the present invention. Effective culture conditions include, but are not limited to, effective media, bioreactor, temperature, pH and oxygen conditions that permit protein production. An effective, medium refers to any medium in which a cell is cultured to produce a protein of the present invention. Such medium typically includes an aqueous medium having assimilable carbon, nitrogen and phosphate sources, and appropriate salts, minerals, metals and other nutrients, such as vitamins. Cells of the present invention can be cultured in conventional fermentation bioreactors, shake flasks, test tubes, microtiter dishes, and petri plates. Culturing can be carried out at a temperature, pH and oxygen content appropriate for a recombinant cell. Such culturing conditions are within the expertise of one of ordinary skill in the art.

Depending on the vector and host system used for production, resultant proteins of the present invention may either remain within the recombinant cell; be secreted into the fermentation medium; be secreted into a space between two cellular membranes, such as the periplasmic space in *E. coli;* or be retained on the outer surface of a cell or viral membrane.

Recovery of proteins of invention can include collecting the whole fermentation medium containing the protein and need not imply additional steps of separation or purification. Proteins of the present invention can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization. Proteins of the present invention are preferably retrieved in substantially pure form thereby allowing for the effective use of the protein as a therapeutic composition. A therapeutic composition for animals, for example, should exhibit no substantial toxicity and preferably should be capable of stimulating the production of antibodies in a treated animal.

One embodiment of the present invention is multivalent vaccine as described in the claims that, when administered to an animal in an effective manner, is capable of protecting that animal from filariasis caused by a filarial nematode. As used herein, the vaccine of the invention is protective in that, when administered to an animal in an effective manner, it is able to treat, ameliorate, and/or prevent disease caused by a filarial nematode including, but not limited to, *W. bancrofti, B. malayi, O. volvulus, L. loa, Mansonella streptocerca, Dracunculus medinensis, M. perstans, M. ozzardi,* and/or *B. timori.* Vaccines of the present invention can be administered to any animal susceptible to such therapy, preferably to mammals, and more preferably to humans, pets such as cats, and economic food animals and/or zoo animals. The preferred animals to protect against elephantiasis include humans.

A of the present invention when administered to the host can develop antibodies that can kill the parasites in the vector in which the filarial nematode develops, such as in a mosquito when they feed the host.

In order to protect an animal from disease caused by a filarial nematode, an immunogenic composition of the present invention is suitable to be administered to the animal in an effective manner such that the composition is capable of protecting that animal from a disease caused by the filarial nematode. Compositions of the present invention can be administered to animals prior to infection in order to prevent infection (*i.e.*, as a preventative vaccine) and/or can be administered to animals after infection in order to treat disease caused by the filarial nematode (*i.e.*, as a therapeutic vaccine).

Compositions of the present invention can be formulated in an excipient that the animal to be treated can tolerate. Examples of such excipients include water, saline, Ringer's solution, dextrose solution, Hank's solution, and other aqueous physiologically balanced salt solutions. Nonaqueous vehicles, such as fixed oils, sesame oil, ethyl oleate, or triglycerides may also be used. Other useful formulations include suspensions containing viscosity enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer and Tris buffer, while examples of preservatives include thimerosal, m- or o-cresol, formalin and benzyl alcohol. Standard formulations can either be liquid injectables or solids which can be taken up in a suitable liquid as a suspension or solution for injection. Thus, in a non-liquid formulation, the excipient can comprise dextrose, human serum albumin, preservatives, etc., to which sterile water or saline can be added prior to administration.

The vaccine of present invention can include an adjuvant. Adjuvants are agents that are capable of enhancing the immune response of an animal to a specific antigen. Suitable adjuvants include, but are not limited to, cytokines, chemokines, and compounds that induce the production of cytokines and chemokines (e.g., granulocyte macrophage colony stimulating factor (GM-CSF), granulocyte colony stimulating factor (G-CSF), macrophage colony stimulating factor (M-CSF), colony stimulating factor (CSF), erythropoietin (EPO), interleukin 2 (IL-2), interleukin-3 (IL-3), interleukin 4 (IL-4), interleukin 5 (IL-5), interleukin 6 (IL-6), interleukin 7 (IL-7), interleukin 8 (IL-8), interleukin 10 (IL-10), interleukin 12 (IL-12), interferon gamma, interferon gamma inducing factor I (IGIF), transforming growth factor beta, RANTES (regulated upon activation, normal T-cell expressed and presumably secreted), macrophage inflammatory proteins (e.g., MIP-1 alpha and MIP-1 beta), and Leishmania elongation initiating factor (LEIF)); bacterial components (e.g., endotoxins, in particular superantigens, exotoxins and cell wall components); toll-like receptor agonists (example TLR4 agonists); aluminum-based salts; calcium-based salts; silica; polynucleotides; toxoids; serum proteins, viral coat proteins; block copolymer adjuvants (*e.g.*, Hunter's TITERMAX adjuvant (VAXCEL, Inc. Norcross, GA), Ribi adjuvants (Ribi ImmunoChem Research, Inc., Hamilton, MT); and saponins and their derivatives (*e.g.,* QUIL A (Superfos Biosector A/S, Denmark). Protein adjuvants of the present invention can be delivered in the form of the protein themselves or of nucleic acid molecules encoding such proteins using the techniques described herein.

A vaccine of present invention can include a carrier. Carriers include compounds that increase the half-life of a therapeutic composition in the treated animal. Suitable carriers include, but are not limited to, polymeric controlled release vehicles, biodegradable implants, liposomes, bacteria, viruses, other cells, oils, esters, and glycols.

The present disclosure also covers a controlled release formulation that is capable of slowly releasing a composition of the present invention into an animal. As used herein, a controlled release formulation may include a composition of the present invention in a controlled release vehicle. Suitable controlled release vehicles include, but are not limited to, biocompatible polymers, other polymeric matrices, capsules, microcapsules, microparticles, bolus preparations, osmotic pumps, diffusion devices, liposomes, lipospheres, and transdermal delivery systems. Other controlled release formulations as disclosed herein include liquids that, upon administration to an animal, form a solid or a gel in situ. Preferred controlled release formulations are biodegradable (*i.e.*, bioerodible).

A preferred controlled release formulation is capable of releasing a vaccine into the blood of the treated animal at a constant rate sufficient to attain therapeutic dose levels of the composition to protect an animal from disease caused by a filarial nematode. The vaccine is preferably released over a period of time ranging from about 1 to about 12 months. A controlled release formulation as disclosed herein is capable of effecting a treatment preferably for at least about 1 month, more preferably for at least about 3 months, even more preferably for at least about 6 months, even more preferably for at least about 9 months, and even more preferably for at least about 12 months.

Vaccines of the present invention can be administered to animals prior to infection in order to prevent infection and/or can be administered to animals after infection in order to treat disease caused by a filarial nematode. For example, proteins, nucleic acids and mixtures thereof can be used as immunotherapeutic agents. Acceptable protocols to administer compositions in an effective manner include individual dose size, number of doses, frequency of dose administration, and mode of administration. Determination of such protocols can be accomplished by those skilled in the art. A suitable single dose is a dose that is capable of protecting an animal from disease when administered one or more times over a suitable time period. For example, a preferred single dose of a protein-based vaccine is from about 1 microgram (pg) to about 10 milligrams (mg) of protein-based vaccine per kilogram body weight of the animal. Booster vaccinations can be administered from about 2 weeks to several years after the original administration. Booster administrations preferably are administered when the immune response of the animal becomes insufficient to protect the animal from disease. A preferred administration schedule is one in which from about 10 μg to about 1 mg of the vaccine per kg body weight of the animal is administered from about one to about two times over a time period of from about 2 weeks to about 12 months. Modes of administration can include, but are not limited to, subcutaneous, intradermal, intravenous, intranasal, oral, transdermal and intramuscular routes.

Wherein the vaccine includes a nucleic acid molecule, the vaccine can be administered to an animal in a fashion to enable expression of that nucleic acid molecule into a protective protein in the animal. Nucleic acid molecules can be delivered to an animal in a variety of methods including, but not limited to, administering a naked (*i.e.*, not packaged in a viral coat or cellular membrane) nucleic acid as a genetic vaccine (*e.g.*, as naked DNA molecules, such as is taught, for example in Wolff, et al. (1990) Science 247:1465-1468); or administering a nucleic acid molecule packaged as a recombinant virus vaccine or as a recombinant cell vaccine (*i.e.*, the nucleic acid molecule is delivered by a viral or cellular vehicle).

A genetic (*i.e.*, naked nucleic acid) vaccine of the present invention includes a nucleic acid molecule of the present invention. A genetic vaccine of the present invention can include one or more nucleic acid molecules of the present invention in the form of, for example, a dicistronic recombinant molecule. Preferred genetic vaccines include at least a portion of a viral genome (*i.e.*, a viral vector). Preferred viral vectors include those based on alphaviruses, poxviruses, adenoviruses, herpesviruses, picornaviruses, and retroviruses, with those based on alphaviruses (such as sindbis or Semliki forest virus), species-specific herpesviruses and poxviruses being particularly preferred. Any suitable transcription control sequence can be used, including those disclosed as suitable for protein production. Particularly preferred transcription control sequences include cytomegalovirus immediate early (preferably in conjunction with Intron-A), Rous sarcoma virus long terminal repeat, and tissue-specific transcription control sequences, as well as transcription control sequences endogenous to viral vectors if viral vectors are used. The incorporation of a "strong" polyadenylation signal is also preferred.

Genetic vaccines of the present invention can be administered in a variety of ways, including intramuscular, subcutaneous, intradermal, transdermal, intranasal and oral routes of administration. Moreover, it is contemplated that the vaccine can be delivered by gene gun, skin patch, electroporation, or nano-based delivery. In this respect, DNA-based and protein-based vaccines can be administered at the same time. A preferred single dose of a genetic vaccine ranges from about 1 nanogram (ng) to about 600 μg, depending on the route of administration and/or method of delivery, as can be determined by those skilled in the art. Suitable delivery methods include, for example, by injection, as drops, aerosolized and/or topically. Genetic vaccines of the present invention can be contained in an aqueous excipient (*e.g.,* phosphate-buffered saline) alone or in a carrier (*e.g.*, lipid-based vehicles).

A recombinant virus vaccine may include a recombinant molecule of the present invention that is packaged in a viral coat and that can be expressed in an animal after administration. Preferably, the recombinant molecule is packaging- or replication-deficient and/or encodes an attenuated virus. A number of recombinant viruses can be used, including, but not limited to, those based on alphaviruses, poxviruses, adenoviruses, herpesviruses, picornaviruses, and retroviruses. Preferred recombinant virus vaccines are those based on alphaviruses (such as Sindbis virus), raccoon poxviruses, species-specific herpesviruses and species-specific poxviruses. Examples of methods to produce and use alphavirus recombinant virus vaccines are disclosed in PCT Publication No. WO 94/17813.

When administered to an animal, such a recombinant virus vaccine infects cells within the immunized animal and directs the production of a protective protein that is capable of protecting the animal from filariasis caused by filarial nematodes. By way of illustration, a single dose of such a recombinant virus vaccine can be from about $1X10^4$ to about $1X10^8$ virus plaque forming units (pfu) per kilogram body weight of the animal. Administration protocols are similar to those described herein for protein-based vaccines, with subcutaneous, intramuscular, intranasal and oral as routes of administration.

A recombinant cell vaccine of as disclosed herein can include recombinant cells of the present invention that express a fusion protein of the present invention. Examples of such recombinant cells include *Salmonella, E. coli, Listeria, Mycobacterium, S. frugiperda,* yeast, (including *Saccharomyces cerevisiae* and *Pichia pastoris),* BHK, CV-1, myoblast G8, COS (*e.g.*, COS-7), Vero, MDCK and CRFK recombinant cells. Recombinant cell vaccines as disclosed herein can be administered in a variety of ways but have the advantage that they can be administered orally, preferably at doses ranging from about $10^8$ to about $10^{12}$ cells per kilogram body weight. Administration protocols are similar to those described herein for protein-based vaccines. Recombinant cell vaccines can include whole cells, cells stripped of cell walls or cell lysates.

As is known in the art, there are three groups of filarial nematodes, classified according to the niche within the body that they occupy: lymphatic filariasis, subcutaneous filariasis, and serous cavity filariasis. Lymphatic filariasis is caused by the worms *W.* bancrofti *B. malayi* and *B. timori.* These worms occupy the lymphatic system, including the lymph nodes, and cause fever, lymphadenitis (swelling of the lymph nodes), lymphangitis (inflammation of the lymphatic vessels in response to infection), and lymphedema (elephantiasis). Subcutaneous filariasis is caused by Loa *loa* (the African eye worm), *Mansonella stretocerca, O. volvulus* and *Dracunculus medinensis.* These worms occupy the subcutaneous layer of the skin, in the fat layer, and present with skin rashes, urticarial papules, and arthritis, as well as hyper- and hypopigmentation macules. *Onchocerca volvulus* manifests itself in the eyes, causing "river blindness." Serous cavity filariasis is caused by the worms *M. perstans* and *M. ozzardi,* which occupy the serous cavity of the abdomen. Serous cavity filariasis presents with symptoms similar to subcutaneous filariasis, in addition to abdominal pain, because these worms are also deep tissue dwellers.

The efficacy of a vaccine of the present invention to protect an animal from filariasis caused by filarial nematodes can be tested in a variety of ways including, but not limited to, detection of protective antibodies (using, for example, fusion proteins of the present invention) or the proteins disclosed herein, detection of cellular immunity within the treated animal, or challenge of the treated animal with the a filarial nematode to determine whether the treated animal is resistant to disease and fails to exhibit one or more signs of disease. Challenge studies can include implantation of chambers including filarial nematode larvae into the treated animal and/or direct administration of larvae to the treated animal. For example therapeutic compositions can be tested in animal models such as mice, jirds (*Meriones unguiculatus*) and/or mastomys (*e.g.*, *Mastomys natalensis*). Such techniques are known to those skilled in the art. The invention is described in greater detail by the following non-limiting examples.

**Comparative Example 1: Small Heat Shock Protein Vaccine**

*Parasites. B. malayi* L3s were obtained from the NIAID/NIH Filariasis Research Reagent Resource Center (FR3) at the University of Georgia, Athens, GA.

*Human Sera Samples.* About 10 ml of blood samples were collected from the following clinical groups of subjects (1) Endemic normal (EN) subjects, these were individuals who were asymptomatic and non-microfilaraemic; (2) asymptomatic microfilaraemic subjects (Mt) who had circulating microfilaria in their blood and were identified by microscopic examination of their night blood smears; (3) Chronic Pathology (CP) patients include those subjects who exhibited lymph edema and other chronic clinical symptoms of filariasis and (4) Non-endemic normal subjects (NEN) who lived in non-endemic areas and had no circulating parasites or antibodies and showed no evidence of any filarial disease. Sera were separated from these blood samples and were stored at -80°C until use.

*Expression and Purification of Recombinant B. malayi Heat Shock Protein.* To produce recombinant *B. malayi* small heat shock protein 12.6 (rBmHSP), the full-length gene sequence was cloned into pRSET-A (with an N-terminal hexahistidine tag) and was transformed into BL21(DE3) containing pLysS (Invitrogen, Carlsbad, CA) to minimize toxicity due to the protein. When absorbance of the cultures reached 0.6 OD value, 1 mM of IPTG (isopropyl thio-d-galactopyranoside) was added to the cultures and incubated for an additional 3 hours to induce gene expression. After lysing the cells, total proteins were separated in a 15% SDS-PAGE to confirm the expression of his-tagged protein. Subsequently, the histidine-tagged recombinant protein was purified using an immobilized cobalt metal affinity column chromatography (Clontech, Mountain View, CA) as per the manufacturer's recommendations. Recombinant protein was then separated in a 15% SDS-PAGE and stained with COOMASSIE brilliant blue R250. A single band was obtained after column purification.

*Three-Dimensional Model of BmHSP.* A three-dimensional model of BmHSP protein was constructed by homology modeling. BLAST sequence homology searches were performed to identify template proteins in the PDB database. Human alpha-crystallin A, a recently crystallized protein, showed significant sequence identity and was therefore chosen as the template for modeling BmHSP. Model building was performed using MODELLER 9v6 (Sali & Blundell (1993) J. Mol. Biol. 234:779-815). The 3-D structure obtained was subsequently validated using PROCHECK program (Laskowski, et al. (1993) J. Appl. Cryst. 26:283-29). The best model predicted by PROCHECK had a score of -0.46 and was chosen for further modeling and for generating the 3-D structure using Rasmol program.

*Analysis of the Structure of BmHSP.* The secondary structure and protein-protein interaction site of BmHSP was predicted at PDBsum and the Predict Protein E-mail server at the European Molecular Biology Laboratory, Heidelberg (Roos, et al. (1995) Parasitol. Today 11:148-150). Motif scanning was carried out via PROSITE pattern analysis to identify the functional motifs in BmHSP. B-cell, T-cell and CTL epitopes in BmHSP sequences were predicted using Immune Epitope Database and Analysis Resource (IEDB).

*Phylogenetic Analysis of BmHSP.* Amino acid sequences of BmHSP were compared with members of other small heat shock family of proteins from different organisms. The following sequences were analyzed. Accession numbers are given in parenthesis. *Aconthocheilonema vitae* (CAA48631); *Archaeoglobus fulgidus* (028308); *Artibeus jamaicensis* (P02482); *Aspergillus fumigatus* (Q4WV00); *Arabidopsis thaliana* (081822); *Artemia persimilis* (DQ310578); Azotobacter *vinelandii* (P96193); *Brugia pahangi* (CAA61152), *Brugia malayi* (AAU04396); *Buchnera aphidicola* (P57640); *Bombyx mori* (AF315318_1); *Bradyrhizobium japonicum* (P70918); *Caenorhabditis elegans* (Q7JP52); *Coccidioides immitis* (Q1E6R4); *Carica papaya* (Q69BI7); *Caenorhabditis remanei* (AAZ42349); *Dictyostelium discoideum* (Q54I91); *Escherichia coli* (ibpA; P0C054); *Escherichia coli* (ibpB; P0C058); *Homo sapiens* (P02489); *Haemonchus contortus* (AAN05752); *Lygodactylus picturatus* (Q6EWI0); *Onchocercara volvulus* (CAA48633), *Ostertagia ostergi* (CAG25499); *Macaca* mulatta (P02488); *Mycobacterium tuberculosis* (P0A5B7); *Mus musculus* (AAA37861); *Nippostrongylus brasiliensis* (BAI81970); *Plasmodium falciparum* (Q8IB02); *Rattus rattus* (CAA42910); *Saccharomyces cerevisiae* (P15992); *Solanum lycopersicum* (082545); *Streptococcus thermophilus* (P80485); *Trichinella spiralis* (ABJ55914); *Trypanosoma brucei* (Q57V53); *Toxoplasma gondii* (Q6DUA8). The alpha-crystallin domain from all sHSP sequences were aligned using ClustalW algorithm and the data set were used to build a phylogenetic tree with the PHYLIP software. The trees were made using the neighbor joining method, with Poisson-corrected amino acid distances.

*Chaperone Assay.* One of the typical characteristics of chaperone is that they can bind to and protect cellular proteins from heat damage. When proteins are exposed to heat damage, they aggregate (thermal aggregation). Chaperones prevent this aggregation. To determine whether BmHSP could prevent thermal aggregation, a citruline synthase (CS) (Sigma, St. Louis, MO) thermal aggregation assay was used. CS was selected because this protein is highly sensitive to heat denaturation. An established method was used (Gnanasekar, et al. (2009) Biochem. Biophys. Res. Commun. 386:333-337). Briefly, 1 μM of CS was exposed to 45°C in the presence or absence of BmHSP (2 μM) suspended in 50 mM of sodium phosphate pH 7.4 buffer containing 100 mM NaCl. BSA was used as a control. CS was incubated with BmHSP at a molar ratio of (1:2) for various time intervals from 0 to 40 minutes. Thermal denaturation (aggregation) was monitored spectrophotometrically at 360 nm.

In *Vitro Peptide Binding Assay for Chaperone Activity.* Another characteristic of heat shock proteins is that they can bind to a variety of proteins. To determine whether BmHSP also possesses this function, CS and another protein, luciferase, were chemically denatured with 6M guanidine hydrochloride according to known methods (Gnanasekar, et al. (2009) *supra).* Native and chemically denatured proteins were then coated onto 96-well plates overnight at 4°C. After washing with PBS, wells were blocked with 3% BSA at room temperature. Following further washing, wells were incubated with his-tagged rBmHSP for 1 hour at 37°C. After washing again with PBS, optimally diluted anti-his-tagged HRP conjugate was added and incubated at 37°C for 1 hour. After final washing, color was developed with ABTS [2,2'-azinobis(3-ethylbenzothiazoline-6-sulfonic acid)] and OD was measured at 405 nm.

*Anti-BmHSP Antibody Levels in Human Sera.* A total of 20 sera samples belonging to different clinical groups such as Mf, CP, EN and NEN were analyzed for the presence and titer of anti-BmHSP IgG antibodies using an indirect ELISA (Cheirmaraj, et al. (1992) J. Trop. Med. Hyg. 95:47-51). Briefly, wells of a 96-well microtiter plate were coated with rBmHSP (1 μg/ml) in carbonate buffer, pH 9.6, overnight at 4°C and blocked with 3% BSA for 1 hour at 37°C. Sera samples were added to the wells and the plates were incubated overnight at 4°C. After washing the wells, HRP-labeled mouse anti-human IgG was added (1:5000) and incubated further for 1 hour at 37°C. Color was developed using ABTS substrate. Absorbance was measured at 405 nm in a microplate reader (BIO-RAD, Hercules, CA). The isotype of anti-BmHSP IgG antibodies in the sera of subjects was also determined using an isotype-specific ELISA. Biotinylated mouse monoclonal antihuman IgG1, IgG2, IgG3 and IgG4 were used as the secondary antibodies and color was developed with avidin-HRP conjugate (Sigma, St. Louis, MO) as the secondary antibodies.

*Cloning of Codon-Optimized BmHSP into pVAX Vector for DNA Vaccine.* Codon-optimized *Bmhsp* genes were cloned into eukaryotic expression vector pVAX (Invitrogen) using insert-specific primers (forward primer, 5'-CGC GGA TCC ATG GAA GAG AAG GTG GTG-3' (SEQ ID NO:1) containing *Bam*HI site and reverse primer, 5'-CCG GAA TTC TCA CTT GTC GTT GGT G-3' (SEQ ID NO:2) containing EcoRI site). PCR parameters were as follows: 94°C of denaturation for 30 seconds, 50°C of primer annealing for 30 seconds, 72°C of primer extension for 30 seconds for 30 cycles; and a final extension of 5 minutes was performed at 72°C. Insert DNA was sequenced to ensure authenticity of the cloned nucleotide sequence on both strands. Plasmids were maintained and propagated in E. *coli* TOP10F' cells. Subsequently, plasmids were purified using endotoxin-free plasmid extraction kit (Qiagen, Hilden, Germany). DNA was analyzed by agarose gel electrophoresis and quantified by spectrophotometry (OD 260/280, ratio> 1.8).

*Immunization of Mice.* Six-week-old male Balb/c mice purchased from Charles River Laboratories were used in these experiments. Humane use of animals in this study and the protocol was approved by the IACUC committee at the College of Medicine, University of Illinois Rockford. Each group was composed of five (5) mice and all mice were immunized intraperitoneally using three different immunization regimens. Group A mice were immunized using a prime-boost regimen. Mice were primed twice at two week intervals with 100 μg of endotoxin-free, codon-optimized pVAX *Bmhsp* DNA suspended in 50 μl volume. Following priming, all mice received two booster doses of 15 μg of rBmHSP

protein (50 μl each) suspended in alum at two weeks interval. Group B mice were immunized with rBmHSP protein alone. These mice received four doses of 15 μg of rBmHSP protein suspended in alum given at two week intervals. Group C mice were immunized with DNA alone. These mice received four doses of 100 μg of pVAX *Bmhsp* DNA given at two week intervals. Group D animals received 100 μg of pVAX vector control and adjuvant at the same interval and remained as negative controls. Blood samples were collected from each mouse before immunization and one month after the last booster dose. After separating the sera, titer of circulating anti-BmHSP IgG antibodies and the respective isotypes were determined. Sera that showed high titer of antibodies against BmHSP were used in the Antibody Dependent Cellular Cytotoxicity (ADCC) assay described herein.

*Anti-BmHSP Antibody Levels in* the *Sera of Mice.* Anti-BmHSP IgG antibody levels in the sera of immunized and control groups of mice were determined using an indirect ELISA (Veerapathran, et al. (2009) PLoS Negl. Trop. Dis. 3:e457). IgG1, IgG2a, IgG2b and IgG3 anti-BmHSP antibody levels were also determined using a mouse antibody isotyping ELISA kit (ThermoFisher Scientific, Rockford, IL). Color was developed with ABTS (2,2'-azinobis (3-ethyl benzothiazoline-6-sulfonic acid) chromogen substrate and the absorbance was measured at 405 nm in an ELISA reader (BIO-RAD).

*Depletion Anti-BmHSP Antibodies from Human and Mice Sera.* Anti-BmHSP antibodies were depleted from pooled sera of EN subjects and immunized mice by incubating the pooled sera with cobalt IMAC resin coupled with his-tagged rBmHSP according to established methods (Veerapathran, et al. (2009) *supra).* Briefly, 1 mg of his-tagged rBmHSP was coupled to 2 ml bed volume of IMAC resin for 2 hours at 37°C. After washing the resin once with 10 ml of PBS (pH.8), 200 μl of pooled sera was added and incubated overnight at 4°C. After incubation, the resin mixture was centrifuged for 2 minutes at 750 rpm and the supernatant was collected. Depletion of anti-BmHSP antibodies in the supernatant was confirmed by ELISA as described herein.

Anti-BmHSP IgG1, anti-BmHSP IgG2a, anti-BmHSP IgG2b, anti-BmHSP IgG3 and anti-BmHSP IgG4 antibodies from pooled sera of EN subjects and pooled sera of immunized mice were depleted using NHS (N-hydroxysuccinimidyl) resin (Thermo fisher scientific). Briefly, 1 μg of respective monoclonal antibodies were coupled to NHS resin column. After washing the resin twice with PBS (pH.8), 100 μl of sera were passed through the column. The flow through was collected as the antibody depleted sera. Depletion of the specific isotype of antibody was confirmed by an isotype-specific ELISA as described herein. After washing the column three times with PBS (pH 7.4), bound antibodies were eluted using Glycine-HCl buffer (pH 2.7) from the resin and the pH was adjusted to 7.4 with 1 M Tris buffer (pH 8). The recovered elute contained the specific antibody as confirmed again by an ELISA. The antibody depleted sera was also reconstituted with the eluted antibodies. An aliquot of depleted sera was reconstituted with the eluted antibodies to its original concentration using values determined by an earlier ELISA on the neat serum samples. Antibody depleted sera, eluted antibodies and reconstituted sera samples were then used in an ADCC assay.

*Antibody-Dependant Cellular Cytotoxicity (ADCC) Assay. In vitro* ADCC assay was performed according to known methods (Chandrasekhar, et al. (1985) Parasite Immunol. 7:633-641). Briefly, ten (10) L3 of *B. malayi* were incubated with $2 \times 10^5$ peritoneal cells (PEC) collected from normal mice, 50 μl of pooled mouse sera samples and 50 μl of RPMI 1640 media in a 96-well culture plate (Thermo Fisher Scientific). After 48 hours of incubation at 37°C and 5% $CO_2$, larval viability was determined at 400X using a light microscope. Larvae that were limpid and damaged were counted as dead. In addition, dead larvae also had clumps of cells adhered to it and were more transparent than live. Larvae that were active, coiled and translucent were counted as live. ADCC was estimated as the percent larval death calculated using the formula:

Number of Dead larvae ÷ Total Number of Larvae x 100.

ADCC assay was also performed with pooled human sera samples as described herein except that the human sera samples were incubated with $2 \times 10^5$ PBMCs collected from normal health subjects and 6-12 *B. malayi* L3 for 48 hours at 37°C and 5% $CO_2$. Larval viability and death was determined as described above.

*Protection Studies in Mice.* Vaccine potential of BmHSP was evaluated in a mouse model of challenge infection. Mice were immunized as described above using prime-boost, DNA alone or protein alone approach. Vector and alum group served as negative controls. Immunized and control animals were challenged using a micropore chamber method as known in the art (Abraham, et al. (1986) Immunology 57:165-169). Briefly, micropore chambers were assembled using 14 x 2 mm PLEXI rings (Millipore Corporations, Bedford, MA) and 5.0 μm nucleopore polycarbonate membranes (Millipore Corporations). The membranes were attached to the PLEXIGLASS rings with cyanoacrylic adhesive and dental cement. The chambers were immersed overnight at 37°C in sterile RPMI medium containing gentamycin and antimycotic solution. Before challenge experiments, 20 live, infective L3s suspended in RPMI 1640 medium supplemented with 15% heat-inactivated fetal calf serum (FCS) were introduced into the micropore chambers and the opening was sealed with dental cement. Micropore chamber containing the L3s were then surgically implanted into the peritoneal cavity of each mice under anesthesia. Aseptic conditions were followed for the surgical procedures. After 48 hours of implantation, animals were sacrificed and the chambers were recovered from peritoneal cavity. Contents of each chamber were emptied and larvae were examined microscopically for adherence of cells and for larval death. Dead and live larvae were identified as described above under ADCC. The percentage of protection was expressed as the number of dead parasites ÷ number of total parasites recovered x 100.

*Splenocyte Proliferation Assay.* Spleens were collected from all mice from the above experiment and single-cell suspension of spleen cells was prepared. Approximately 2 x $10^5$ cells/well suspended in complete RPMI 1640 medium supplemented with 10% heat-inactivated FCS were incubated at 37°C and 5% $CO_2$ for 72 hours with rBmHSP (1 μg/ml), ConA (1 μg/ml) or with medium alone. After incubation, cell proliferation was determined using cell counting kit (CCK-8) purchased from Dojindo Molecular Technologies, Inc. (Gaithersburg, MD). Stimulation index of spleen cell proliferation was calculated using the formula: Absorbance of stimulated cells ÷ Absorbance of unstimulated cells.

*Cytokine Analysis.* Spleen cells from immunized and control mice were cultured at 37°C and 5% $CO_2$ for 72 hours with rBmHSP (1 μg/ml), ConA (1 μg/ml) or with medium alone as described above. After 72 hours, culture supernatants and cell pellets were collected separately for cytokines analysis. For measuring cytokine mRNA, cell pellets were suspended in TRIZOL reagent (GIBCO-BRL, Life technologies, Carlsbad, CA) and total RNA was extracted as per the manufacturer's instructions. After ethanol washes, RNA pellets were dissolved in RNAse-free water (Sigma) and treated with DNase I before determining total RNA concentration using a Beckman spectrophotometer at 260 nm. Reverse transcription of total RNA was performed using first strand cDNA synthesis kit (SABiosciences, Frederick, MD) as per manufacturer's recommendations. Relative quantification of the expression of genes of interest was measured in an Applied BioSystem 7300 real-time PCR machine (Applied BioSystems, Foster City, CA). PCR amplifications were performed with the LightCycler-DNA SYBR Green mix (SAbiosciences). The reaction was performed using the following PCR conditions: 15 minutes activation step at 95°C for one cycle, 15 seconds denaturation step at 95°C, annealing of primers for 20 seconds at 50°C and elongation step for 15 seconds at 72°C. DNA was amplified for 50 cycles. The fluorescent DNA binding dye SYBR Green was monitored. RT-PCR data array set was generated and analyzed using SABiosciences web-based data analysis system.

Culture supernatants were then collected from splenocyte cultures 72 hours after incubation with rBmHSP (1 μg/ml), ConA (1 μg/ml) or with medium alone. Secreted levels of IL-2, IL-4, IFN-γ and IL-10 protein in the culture supernatants were determined using a sandwich ELISA kit purchased from ThermoFisher Scientific. Concentration of each cytokine was determined from a standard curve plotted using recombinant mouse IL-2, IL-4, IFN-γ or IL-10.

*Statistical Analysis.* Statistical analysis was performed using XL STAT software v.7.5.2 (Kovach Computing Services, Anglesey, UK). Statistical significance between comparable groups was estimated using appropriate non-parametric tests, with the level of significance set at $p<0.05$.

*Expression of Recombinant BmHSP12.6 (BmHSP).* BmHSP was cloned in pRSET A vector and was expressed as a histidine-tagged (his-tagged) fusion protein in E. *coli* BL21 (DE3)PLysS. Recombinant BmHSP protein was subsequently purified using IMAC column. The molecular mass of the purified recombinant his-tag fusion protein was found to be approximately 18 kDa. The column-purified recombinant protein appeared as a single band in SDS-PAGE.

*Predicted Three-Dimensional Structure of BmHSP.* Amino acid sequences of the human alpha crystalline A chain share 42% similarity with BmHSP. Since crystal structure of the human alpha crystalline A chain is already available, this was used that as a template to model the putative structure of BmHSP using the Modeller 9v6 program. PROCHECK analysis was used to select the best model that showed a score of -0.41 compared to the template (Laskowski, et al. (1993) J. Appl. Cryst. 26:283-29). A Ramachandran plot analysis was also performed on the BmHSP sequence. These analyses showed that 92% of residues were in the most favorable region with no steric hindrance. About 6.7% residues were found in the additional allowed region. Models that showed over 90% residues in the most favored regions were predicted as the most ideal three dimensional model as predicted by the Ramachandran plot (Balazs, et al. (2001) Protein Eng. Des. Sci. 14:875-880). Secondary structure prediction analysis was also performed on BmHSP protein using PDBsum server at EMBL. This analysis showed that each alpha-crystalline domain of BmHSP monomer had an immunoglobulin core composed of seven β-strands arranged in two anti-parallel sheets. The secondary structure prediction of BmHSP showed two sheets, four beta hairpins, one beta bulge, seven strands, two helices, seven beta turns and one gamma turn in the structure of BmHSP.

Previous studies showed that BmHSP binds to human IL-10 receptor I α chain (Gnanasekar, et al. (2008) Mol. Biochem. Parasit. 159:98-103). To identify the IL-10 receptor binding site on BmHSP, a predictive protein-protein interaction analysis was performed (Ofran & Rost (2007) Bioinformatics 23:e13-e16). Results from the prediction analysis showed that the N-terminal fragment of BmHSP (amino acids from Metl to Asn26) had a strong protein-protein interaction region. Further sequence analysis of this region showed that the amino acid sequences from Val5 to Glu42 had significant sequence identity to human IL-10R binding region of human IL-10. These findings confirm that the N-terminal region of BmHSP may be involved in the binding of BmHSP to human IL-10 receptor I α chain.

*Motif and Phylogenetic Analysis on BmHSP.* Motif analysis performed at PROSITE showed several putative post-translation modification sites such as N-glycosylation sites (residues 11 to 14 and 98 to 101), protein kinase-c phosphorylation sites (residues 83 to 85 and 100 to 102), casein kinase II phosphorylation sites (residues 68 to 71 and 88 to 91) and N-myristylation sites (residues 40 to 45) on BmHSP. Similar motifs were also observed in human IL-10 further indicating that BmHSP may mimic human IL-10 function (Gnansekar, et al. (2008) *supra).* Epitope mapping on BmHSP revealed the presence of B-cell, T-cell and CTL epitope regions, indicating that BmHSP is potentially a highly immunogenic protein (Table 2).

TABLE 2

| Epitope Predicted | Position of Epitope in the Amino Acid Sequence | Peptide Sequence | SEQ ID NO: |
|---|---|---|---|
| B-Cell Epitopes | 12 - 23 | WSAEQWDWPLQH | 3 |
| | 26 - 35 | EVIKTNTNDK | 4 |
| | 67 - 74 | SRAEHYGE | 5 |
| | 84 - 94 | KLPSDVDTKTL | 6 |
| T-Cell Epitopes | 45 - 53 | FTPKEIEVK | 7 |
| | 50 - 57 | IEVKVAGD | 8 |
| | 38 - 45 | VGLDASFF | 9 |
| | 39 - 47 | GLDASFFTP | 10 |
| | 52 - 60 | VKVAGDNLV | 11 |
| | 84 - 92 | KLPSDVDTK | 12 |
| | 92 - 100 | KTLTSNLTK | 13 |
| | 27 - 35 | VIKTNTNDK | 14 |
| | 90 - 98 | DTKTLTSNL | 15 |
| | 44 - 52 | FFTPKEIEV | 16 |
| | 38 - 46 | VGLDASFFT | 17 |
| | 100 - 108 | KRGHLVIAA | 18 |
| | 73 - 81 | GEIKREISR | 19 |
| | 43 - 51 | SFFTPKEIE | 20 |
| CTL Epitopes | 78 - 86 | REISRTYKL | 21 |
| | 74 - 82 | GEIKREISR | 22 |
| | 70 - 78 | AEHYGEIKR | 23 |

A phylogenetic analysis performed using representative sHSP sequences from different groups of organisms showed that BmHSP, *C. elegans* HSP and *C. remani* HSP form a monophyletic group, separated from the other groups of organisms.

*BmHSP is a Chaperone.* Most of the heat shock proteins reported to date have chaperone function. To determine whether BmHSP also has similar chaperone function, a thermal aggregation reaction was performed using a model substrate, Citrulline synthase (CS). Incubation of CS at 42°C resulted in unfolding of the protein and subsequent aggregation within 10 minutes. Addition of BmHSP to CS protein (at a molar ratio of 1:2), before the heat treatment, significantly (P<0.01) inhibited the thermal aggregation of CS protein. A non-chaperone control protein, BSA, had no effect on the heat-induced aggregation of CS protein.

Another function of chaperone proteins is that they can specifically bind to denatured proteins. To determine whether BmHSP can specifically bind to denatured proteins, rBmHSP was incubated with native and denatured CS or native and denatured luciferase substrates. These studies showed that rBmHSP preferentially bound to denatured protein substrates compared to native or control protein. These findings thus confirmed that BmHSP can act as a molecular chaperone potentially protecting the parasite cellular proteins from the damaging effects of the host.

Antibody *Responses in Human.* The results presented herein indicate that BmHSP has several T-cell and B-cell epitopes. Therefore, it was evaluated whether filariasis-infected individuals carry antibodies to BmHSP. Accordingly, the titer of anti-BmHSP IgG antibodies in the sera of EN, CP, Mf and NEN subjects was measured. The results showed that the EN subjects had the highest levels of anti-BmHSP antibodies (p<0.001). Subsequent isotype analysis of the IgG antibodies showed that compared to the infected groups (Mf and CP) of individuals, sera from EN subjects had high titers of IgG1 and IgG3 anti-BmHSP antibodies. Mf carriers had only significant levels of anti-BmHSP IgG2 antibodies in their sera. Similarly, CP individuals had only significant levels of anti-BmHSP IgG4 antibodies in their sera. Anti-BmHSP IgG1 and IgG3 levels were very low in the sera of these Mf and CP individuals. Anti-BmHSP antibodies were not detectable in the sera of NEN subjects.

*Results of ADCC Assay.* Since antibodies to BmHSP were present in all infected groups of individuals (Mf and CP) and EN subjects, it was determined whether these antibodies were functional. Using an antibody-dependent cell cytotoxicity assay, it was tested if anti-BmHSP12.6 IgG antibodies had any protective function against B. *malayi.* These studies showed that pooled EN sera promoted adherence of PBMC's to L3 and induced significant (77.37%) death of B. *malayi* L3s *in vitro* (Table 3), whereas, pooled sera from Mf and CP failed to participate in the ADCC function. These findings indicated that EN sera have antiparasitic activities. To determine if this function is associated with antibodies, antibody depletion studies were performed. Depletion of anti-BmHSP antibodies from EN sera resulted in significant reduction (21.42%) in larval death (Table 3) confirming that anti-BmHSP antibodies in the sera of EN subjects, but not Mf or CP subjects, participate in larval killing.

TABLE 3

| Groups | Dead L3 | Live L3 | Total L3 | % Larval Death (Mean±SD*) |
|---|---|---|---|---|
| Endemic Normal (EN) sera | 5 | 1 | 6 | 77.37±8.41 |
| | 5 | 2 | 7 | |
| EN sera depleted of anti-rBmHSP antibodies | 2 | 5 | 7 | 21.42±10.12 |
| | 1 | 6 | 7 | |
| Non-Endemic Normal (NEN) sera | 1 | 5 | 6 | '19.44±3.92 |
| | 2 | 7 | 9 | |
| *Values represent mean ± SD of three wells. | | | | |

Further depletion studies showed that the antiparasitic effect of anti-BmHSP antibodies was associated with IgG1 isotype of antibodies. Depletion of IgG1 antibodies from EN sera significantly (40%) inhibited the ADCC function (Table 4). Reconstitution of anti-BmHSP antibody depleted EN sera with eluted anti-BmHSP IgG1 antibodies regained the ADCC function (Table 3). These findings thus indicated that anti-BmHSP IgG1 antibodies are critical for ADCC function.

TABLE 4

| EN Sera | | % Larval Death |
|---|---|---|
| Neat Sera | | 72 |
| Depleted of: | IgG1 | 40 |
| | IgG2 | 71.43 |
| | IgG3 | 60 |
| | IgG4 | 62.5 |
| Reconstituted with: | IgG1 | 70 |
| | IgG2 | 69.23 |
| | IgG3 | 66.67 |
| | IgG4 | 54.55 |
| Values represent mean of three wells. | | |

*Antibody Responses in Mice.* Mice immunized with rBmHSP developed significant levels of anti-BmHSP IgG antibodies. More specifically, prime-boost vaccine regimen induced significantly higher titer of IgG antibodies compared to DNA vaccine alone group ($p < 0.05$). However, rBmHSP protein vaccine induced the highest IgG antibody titer. Analysis of the isotype of anti-BmHSP IgG antibodies showed that predominantly IgG1, IgG2a and IgG2b anti-BmHSP antibodies were present in the sera of vaccinated animals. The ADCC assay was also performed with mouse sera. These studies showed that sera from BmHSP-vaccinated mice promoted adherence of peritoneal exudate cells to L3 and participated in ADCC function (83.02% larval killing) compared to control sera (13%) (p<0.002) (Table 5).

TABLE 5

| Immunization Regimen | % Larval Death |
|---|---|
| *Bmhsp* DNA prime and rBmHSP protein boost | 83.02±3.62 |
| *Bmhsp* DNA | 43.7±8.12 |
| rBmHSP protein | 55.08±1.15 |
| pVAX & alum control | 13±2.35 |
| Values represent mean ± SD of three wells. | |

Similar to human sera, individual isotype of IgG antibodies were depleted from the sera of vaccinated mice to determine the isotype of anti-BmHSP antibodies that participate in the ADCC function. Results from these studies showed that, similar to that observed with EN sera, anti-BmHSP IgG1 antibodies were involved in ADCC-mediated killing of L3 in mice as well (Table 6).

TABLE 6

| Immunized Mice Sera | | % Larval Death |
|---|---|---|
| Neat Sera of BmHSP prime-boost | | 80.16 |
| Depleted of: | IgG1 | 37 |
| | IgG2a | 72 |
| | IgG2b | 71 |
| | IgG3 | 80 |
| Reconstituted with: | IgG1 | 80 |
| | IgG2a | 63 |
| | IgG2b | 87 |
| | IgG3 | 71 |
| Values represent mean of three wells. | | |

*Vaccine Potential of BmHSP in Mice.* Vaccine potential of BmHSP was assessed in Balb/c mice using a micropore chamber method. Results showed that mice immunized using the prime-boost vaccination regimen and protein vaccine of BmHSP exhibited nearly 72% and 58% mortality, respectively, of L3s implanted into the peritoneal cavity of the immunized mice (Table 7). While chambers implanted in the control groups of animals showed only 7% mortality of the parasite, the difference between the protection of control group of mice and vaccinated mice was significant (P<0.001). On the other hand, mice immunized by DNA vaccine alone induced only 31% protection. Thus, the prime-boost vaccination regimen appeared to be highly efficient in conferring vaccine-induced protection against a challenge infection compared to DNA alone or protein alone immunization protocols.

TABLE 7

| Immunization Regimen | % Larval Death |
|---|---|
| *Bmhsp* DNA prime and rBmHSP protein boost | 72±10.22 |
| *Bmhsp* DNA | 31±5.23 |
| rBmHSP12.6 protein | 58±7.76 |
| pVAX & alum control | 7±5.2 |
| Values represent mean ± SD. N=5. Data is from one of two similar experiments showing comparable results. | |

*Immune Responses in BmHSP Vaccinated Mice.* To determine cellular immune responses to BmHSP in the vaccinated mice, spleen cells collected from vaccinated and control mice were cultured in the presence of rBmHSP protein and their proliferative responses and cytokine profiles were evaluated. Proliferative response of spleen cells from animals immunized with the prime-boost vaccine regimen was significantly (P>0.05) higher (stimulation index of 3.35 ± 0.176) compared to rBmHSP protein alone vaccination group (stimulation index of 2.22 ± 0.018) or *Bmhsp* DNA vaccination alone group (stimulation index of 3.53 ± 0.102). Spleen cells from the control group of animals failed to proliferate in response to rBmHSP (stimulation index of 0.98 ± 0.013) and was similar to media alone controls. Since the spleen cells from vaccinated animals were proliferating significantly to recall response to rBmHSP, levels of cytokines in the culture supernatants were measured. These results showed that IFN-γ was the predominant cytokine secreted by spleen cells from vaccinated animals at 72 hours after stimulation with rBmHSP. A real time-PCR cytokine gene array was performed on mRNA collected from the spleen cells stimulated with rBmHSP. These results showed that both Th1 (IFN-γ, CD-28, IL-12, IL-2) and Th2 (IL-4, IL-5, IL-1R) cytokine genes were significantly increased in vaccinated animals.

**Comparative Example 2: rBmALT-2+rBmHSP Multivalent Vaccine**

*Parasite. Brugia malayi* L3s were obtained from the NIAID/NIH Filariasis Research Reagent Resource Center (FR3) at the University of Georgia, Athens, GA.

*Construction of Monovalent and Multivalent DNA Vaccines.* Monovalent DNA vaccine was composed of Bmhsp or Bmalt-2 in pVAX1 vector. To prepare the monovalent vaccine, codon optimized Bmhsp or Bmalt-2 genes were cloned into the eukaryotic expression vector pVAX1 (Invitrogen, Carlsbad, CA) using insert-specific primers (Gnanasekar, et al. (2004) *supra).* The multivalent vaccine was composed of Bmhsp and Bmalt-2 genes in the same pVAX1 vector. Codon optimized Bmhsp gene was first cloned into pVAX1 vector with no stop codon in the reverse primer (5'-CCG GAA TTC TCA CTT GTC GTT GGT G-3'; SEQ ID NO:24) but contained a PstI site. Codon optimized Bmalt-2 gene was then inserted into this clone using gene specific primers (Gnanasekar, et al. (2004) *supra*). PCR parameters for all the three constructs were: 94°C denaturation for 30 seconds, 50°C primer annealing for 30 seconds, 72°C primer extension for 30 seconds for 30 cycles; a final extension of 5 minutes was performed at 72°C. Insert DNA was finally sequenced to ensure authenticity of the cloned nucleotide sequence on both strands. Plasmids were maintained and propagated in E. *coli* TOP10F' cells. Plasmids were purified using endotoxin-free plasmid extraction kit (Qiagen, Valencia, CA). DNA was analyzed by agarose gel electrophoresis and quantified in a spectrophotometer (OD 260/280, ratio >1.8).

*Expression and Purification* of *Recombinant Proteins.* All the genes were cloned in pRSET-A vector (with an N-terminal hexahistidine tag) to produce recombinant proteins. Bmhsp and Bmalt-2 constructs were transformed into BL21(DE3) containing pLysS E. *coli* host (Invitrogen) to minimize toxicity due to the protein. When absorbance of the cultures reached 0.6 OD value, 1 mM of IPTG (isopropyl thio-d-galacto pyranoside) was added to the cultures and incubated for an additional 3 hours to induce the gene expression. After lysing the cells, total proteins were separated in 15% and 12% SDS-PAGE to confirm the expression of his-tag recombinant BmHSP (rBmHSP) and rBmALT-2 proteins. The recombinant proteins were then purified using an immobilized cobalt metal affinity column chromatography (Clontech, Mountain View, CA) as per the manufacturer's recommendations. Recombinant proteins were then separated in SDS-PAGE and stained with COOMASSIE brilliant blue R250 and silver stain. These studies showed that a single band was obtained after column purification. Endotoxins if any in the recombinant preparations were removed by passing the recombinant proteins through polymyxin B affinity columns (Thermo Fisher Scientific, Rockford, IL) and the levels of endotoxin in the final preparations were determined using an E-TOXATE kit (Sigma, St Louis, MO) as per manufacturer's instructions. Endotoxin levels were below detection limits in these recombinant protein preparations.

*Immunization of Mice.* Six-weeks old male Balb/c mice purchased from Charles River Laboratories were used in these experiments. Humane use of animals in this study and the protocol was approved by the IACUC committee at the College of Medicine, University of Illinois Rockford. Mice were divided into four (4) groups of five (5) animals each. All mice were immunized subcutaneously using a DNA prime - protein boost vaccine regimen. All experimental groups of mice were primed with two injections of endotoxin-free codon optimized DNA given in 50 μl volume and boosted with two doses of recombinant proteins suspended in alum (50 μl each) given at two weeks interval.

Group A mice were primed with 100 μg of pVAXBmhsp and boosted with 15 μg of rBmHSP; Group B mice were primed with 100 μg of pVAX Bmalt-2 and boosted with 15 μg of rBmALT-2; Group C mice were primed with 100 μg of pVAXBmhsp/Bmalt-2 DNA and boosted with 15 μg of rBmHSP and 15 μg of rBmALT-2. Group D mice received 100 μg of pVAX1 vector plus 50 μl of alum and served as controls. Blood samples were collected from each mouse before immunization and one month after the last booster dose. Sera were separated and stored at -80°C.

*Evaluation of Antibody Responses in Mice.* Levels of anti-BmHSP and anti-BmALT-2 antibodies were measured in the sera of immunized and control groups of mice using an indirect ELISA according to established methods (Veerapathran, et al. (2009) *supra;* Gnanasekar, et al. (2004) *supra).* Briefly, wells of 96-well microtiter plates were coated with rBmHSP, rBmALT-2 or rBmHSP (1 μg/ml) in carbonate buffer (pH 9.6) overnight at 4°C. After washing the wells, unbound sites were blocked with 3% BSA for 1 hour at 37°C. Diluted sera samples were then added to the wells and incubated further overnight at 4°C. After washing the wells, HRP-labelled rabbit anti-mouse IgG was added (1:5000) and incubated further for 1 hour at 37°C. Color was developed using ABTS (2, 2'-azinobis (3-ethylbenzothiazoline-6-sulfonic acid)) substrate. Absorbance was measured at 405 nm in a microplate reader (BIO-RAD, Hercules, CA).

*Protection Studies in Mice.* Vaccine potential of the monovalent and multivalent vaccine formulations were then evaluated in a mice model. Mice were immunized as described above using the prime boost approach. Vector plus alum group served as negative controls. Immunized and control animals were challenged using a micropore chamber method known in the art (Abraham, et al. (1989) Am. J. Trop. Med. Hyg. 40(6) : 598-604). Briefly, micropore chambers were assembled using 14 x 2 mm PLEXIGLASS rings (Millipore Corporations, Bedford, MA) and 5.0 μm nucleopore polycarbonate membranes (Millipore Corporations) that were attached to the PLEXIGLASS rings with cyanoacrylic adhesive and dental cement. The chambers were immersed overnight at 37°C in sterile RPMI medium containing gentamycin and antimycotic solution. Before challenge experiments, 20 live infective L3s suspended in RPMI1640 medium supplemented with 15% heat inactivated fetal calf serum (FCS), were introduced into the micropore chambers and the opening was sealed with dental cement. Micropore chamber containing the L3s were then surgically implanted into the peritoneal cavity of each mice under anaesthesia. Aseptic conditions were followed for the surgical procedures. After 48 hours of implantation, animals were sacrificed and the chambers were recovered from peritoneal cavity. Contents of each chamber were emptied and larvae were examined microscopically for adherence of cells and for larval death. Larval viability was determined microscopically at 100 x. The percentage of protection was expressed as the number of dead parasites ÷ number of total parasites recovered x 100.

*Cytokine Analysis in Mice.* The percent of rBmHSP and rBmALT-2 specific interferon-γ (IFN-γ) and interleukin-4 (IL-4) secreting cells were determined in the spleen of control and vaccinated mice using an ELISPOT assay. Briefly, MILLIPORE MultiScreen HTS Filter plates were coated with monoclonal rat anti mouse IFN-γ or monoclonal rat anti-mouse IL-4 antibodies (BD Pharmigen, San Diego, CA) at a concentration of 10 μg/ml in PBS buffer. After washing the plates, non-specific sites were blocked by incubating the wells in complete RPMI with 10% fetal calf serum for one hour at room temperature. Approximately 3 x $10^6$ spleen cells suspended in complete RPMI1640 medium supplemented with 10% heat inactivated FBS were then added to each wells. Cells were stimulated with rBmHSP or rBmALT-2 (1 μg/ml). Unstimulated cells served as controls. Forty-eight hours after incubation at 37°C in humidified 5% $CO_2$, plates were washed and further incubated for 1 hour at room temperature with 2 μg of biotinylated rat anti-mouse IFN-γ or biotinylated rat anti-mouse IL-4 antibody (BD Pharmigen). After washing the plates, streptavidin-conjugated horseradish peroxidase (Thermo Fisher Scientific) was added (1:800) to each well and incubated at room temperature for one hour. Plates were washed and color developed using DAB substrate (Thermo Fisher Scientific). Total numbers of spots were counted under a dissection microscope.

*Statistical Analysis.* Statistical analysis was performed using XL STAT software v.7.5.2 (Kovach Computing Services, Anglesey, UK). Statistical significance between comparable groups was estimated using appropriate non-parametric tests, with the level of significance set at $p<0.05$.

*Antibody Responses in Mice.* It was first determined whether the multivalent vaccine could elicit significant antibodies against each of the antigenic components. Previous studies have shown that mice similarly vaccinated with *B. malayi* antigens elicited significant *host* protective IgG antibodies (Veerapathran, et al. (2009) *supra).* Therefore, IgG antibody titers were analyzed. The results of this analysis indicated that the monovalent immunization with Bmhsp + rBmHSP and Bmalt-2 + rBmALT-2 elicited significant ($p< 0.005$) titers of anti-BmHSp and anti-BmALT-2 IgG antibodies (Figure 1). The multivalent vaccine also elicited significant IgG antibody titers. Following multivalent vaccine, the mice produced IgG antibodies against both BmHSP and BmALT-2 equally, suggesting that the antigens do not interfere or compete for dominance. An interesting finding was that the multivalent vaccine elicited 1.5- to 1.75-fold higher ($p< 0.005$) titers of IgG antibodies compared to the monovalent vaccine (Figure 1). These finding indicated that the two antigens in the multivalent formulation can act synergistically by increasing the vaccine-induced antibody responses against each antigens in the vaccinated mice. The findings also indicated that combining these two antigens in the vaccine formulation has a great advantage. Given the robust IgG antibody responses induced following vaccination, it is also possible that the concentration of the component antigens in the multivalent preparation can be reduced.

Multivalent *Vaccine Induces Significant Protection in Mice.* The results herein showed that significant IgG antibodies were elicited following vaccination with monovalent and multivalent vaccine preparations. To test if the immune responses elicited following vaccination were protective, vaccinated animals were challenged with live third stage infective larvae (L3) of *B. malayi.* Since the parasites do not reach maturity in these animals, a *better* recovery of worms is obtained if the parasites are surgically implanted into the animals. A standard micropore chamber challenge method (Abraham, et al. (1989) *supra*). These studies showed that close to 61% protection could be achieved in mice immunized with a monovalent vaccine (Table 8). This was highly significant ($p <0.001$) compared to negative controls. This finding also showed that rBmHSP and rBmALT-2 are of use in vaccines for lymphatic filariasis. Challenge experiments in mice immunized with multivalent vaccine showed that significantly ($p <0.005$) higher protection could be achieved compared to monovalent vaccination (Table 8). These findings also clearly correlated with the higher IgG antibody titer in these animals and support the above finding that rBmALT-2 and rBmHSP can synergistically enhance the protective immune responses in vaccinated animals when given as a prime boost regimen (Table 8).

TABLE 8

| Vaccination regimen | Percent Larval Death[a] | Groups |
|---|---|---|
| *Bmhsp* DNA prime and rBmHSP protein boost | 61 ± 4.24 | Monovalent |
| *Bmalt-2* DNA prime and rBmALT-2 protein boost | 76 ± 8.21 | Monovalent |
| *Bmhsp+Bmalt-2* prime and rBmHSP and rBmALT-2 protein boost | 90 ± 7.53 | Multivalent |
| pVAX plus alum control | 22 ± 10.41 | Control |
| [a]Values are mean + SD. N=5. Data is from one of two similar experiments showing comparable results. | | |

To further demonstrate efficacy, mice were immunized with various prime-boost combinations. As shown in Figure 3, 100% protection can be achieved in mice following immunization with HAT hybrid protein or after prime boost immunization with HAT hybrid DNA and HAT hybrid protein.

*Cytokine Responses.* The immunological characteristics of the protective responses in vaccinated mice were determined by evaluating the secreted cytokine responses of spleen cells in response to the vaccine antigens. When spleen cells were stimulated with rBmHSP or rBmALT there was significant antigen-specific proliferation of spleen cells suggesting a strong recall cellular response to the antigens. To identify the cytokine profile of these antigen-responding cells, the IFN-$\gamma$ and IL-4 secreting cells were counted using an ELISPOT assay. Results from these studies showed that spleen cells from mice vaccinated with multivalent vaccine were predominantly secreting IL-4 (Figure 2). The numbers of IFN-$\gamma$ secreting cells were very low. Overall, these findings indicated that vaccine-induced protection was largely mediated by Th2 type responses.

**Comparative Example 3: BmVal-1+BmALT-2 Multivalent Vaccine**

*Sera.* Sera samples used in this study were from archived samples stored at the Mahatma Gandhi Institute of Medical Sciences, Sevagram, India. These samples were collected as part of epidemiological surveys in and around Wardha, an area endemic for lymphatic filariasis.

No demographic data was available to this study except that the sera samples were classified into microfilaremic (MF), chronic pathology (CP) or Endemic normals (EN) based on the detection of circulating parasites, parasite antigens or by evaluating clinical symptoms of lymphatic filariasis. Circulating microfilariae were detected in the blood of subjects according to known methods (Haslbeck, et al. (2005) Nat. Struct. Mol. Biol. 12:842-846; Yoo, et al. (2005) Biotechnol. Lett. 27:443-448). The presence of circulating antigen was detected using an Og4C3 kit and a WbSXP-based enzyme-linked immunosorbent assay (ELISA). Subjects with no circulating antigen or microfilariae were classified as EN, whereas subjects with circulating microfilariae and/or circulating antigen, as detected by ELISA, were considered as MF. Subjects showing lymphedema and other visible clinical symptoms of filariasis were grouped into CP. Control non-endemic normal (NEN) sera were collected at the University of Illinois Clinic at Rockford, IL.

*Parasites. Brugia malayi* L3s were obtained from the NIAID/NIH Filariasis Research Reagent Resource Center (FR3) at the University of Georgia, Athens, GA.

*Construction of Monovalent and Multivalent DNA Vaccines.* To prepare monovalent vaccine, codon optimized *BmVAL-1* (Ace: AF042088) or *Bmalt-2* (Ace: U84723) genes were cloned into the eukaryotic expression vector pVAX1 (Invitrogen, Carlsbad, CA) using insert-specific primers (Yoo, et al. (2005) *supra*; Huang, et al. (2005) Immunol. Lett. 101:71-80). To prepare multivalent vaccine, codon-optimized *BmVAL-l* gene was first cloned into pVAX1 vector with no stop codon using already published primer sequences with a PstI site. Codon-optimized *Bmalt-2* gene was then inserted into this clone using gene-specific primers. PCR parameters for all the constructs were: 94°C denaturation for 30 seconds, 50°C primer annealing for 30 seconds, 72°C primer extension for 30 seconds for 30 cycles; and a final extension of 5 minutes was performed at 72°C. Insert DNA was sequenced to ensure authenticity of the cloned nucleotide sequence on both strands. Plasmids were maintained and propagated in *E. coli* TOP10F' cells. Plasmids were purified using endotoxin-free plasmid extraction kit (Qiagen, Valencia, CA). DNA was analyzed by agarose gel electrophoresis and quantified in a spectrophotometer (OD 260/280, ratio > 1.8).

*Expression and Purification of Recombinant Proteins.* Recombinant BmVAL-L and rBmALT-2 were expressed in pRSET-A vector and purified using an immobilized cobalt metal affinity column chromatography according to published methods (Norimine, et al. (2004) Infect. Immun. 72:1096-1106; Shinnick, et al. (1988) Infect. Immun. 56:446-451). Endotoxin in the recombinant preparations were removed by passing the recombinant proteins through polymyxin B affinity columns (Thermo Fisher Scientific, Rockford, IL) and the levels of endotoxin in the final preparations were determined using an E-TOXATE kit (Sigma, St Louis, MO) as per manufacturer's instructions. Endotoxin levels in the final preparations (0.005 EU/ml) were below detection limits in these recombinant protein preparations.

*Immunoreactivity of Human Sera.* To determine if the human sera samples carried antibodies against BmVAL-1 or BmALT-2, an ELISA was performed (Haslbeck, et al. (2005) *supra;* Yoo, et al. (2005) *supra).* For isotype-specific ELISA, alkaline phosphatase-conjugated goat anti-human IgG1, anti-human IgG2, anti-human IgG3, and anti-human IgG4 antibodies (Sigma) were used as the secondary antibodies.

*Immunization Protocol for Mice and Jirds.* Six-week old male Balb/c mice and 35-40 gm outbred male mangolian gerbils (jirds) purchased from Charles River Laboratories (Wilmington, MA) were used in these experiments. Animals were treated as per the guidelines in the Guide for the Care and Use of Laboratory Animals. Two different animal models were used because *B. malayi* parasite does not mature into adults in mouse, so vaccine-induced protection against the L3 stages can be evaluated in the mouse model. In addition, significant immunological parameters can be measured in mice. Conversely, *B. malayi* parasite develops into mature adult worms in jirds. Therefore, vaccine-induced protection can be evaluated against adult worm establishment in jirds.

Three sets of experiments were performed: (1) monovalent BmVAL-1 vaccination, (2) monovalent BmALT-2 vaccination and (3) multivalent mVAL-1/BmALT-2 vaccination. Each experimental set had four groups (a) DNA prime plus DNA boost (homologous), (b) protein prime plus protein boost (homologous), (c) DNA prime plus protein boost (heterologous) and pVAX plus alum controls. Each group included ten (10) animals each. All animals were immunized subcutaneously with codon-optimized DNA (100 μg) in 50 μl volume or with recombinant protein (150 μg) plus alum in 50 μl volume. Control group received 100 μg of pVAX1 blank vector or 50 μl of alum. Blood samples were collected at frequent intervals, sera separated and stored at -80°C. The protocol used for immunizing mice and jirds was as follows. Animals were prebled and given a first dose on day 0. A second dose was administered on day 14 and subsequently bled. Third and fourth doses were administered on days 28 and 42, respectively, and the animals were subsequently bled. Mice were challenged on day 56 and protection was determined on day 58. Jirds were challenged on day 60 and protection was determined on day 155.

*Protection Studies in Mice.* Challenge studies were conducted in mice by surgically implanting twenty live, infective *B. malayi* L3s into the peritoneal cavity in a micropore chamber (Veerapathran, et al. (2009) *supra;* Abraham, et al. (1988) *supra).* Aseptic conditions were followed for the surgical procedures. Forty-eight hours after implantation, chambers were recovered from the peritoneal cavity and viability of the larvae was determined under a light microscope. The percentage of protection was expressed as the number of dead parasites + number of total parasites recovered x 100.

*Splenocyte Proliferation and Cytokine Assays.* Single-cell suspension of spleen cells (0.5 x $10^6$ cells per well suspended in 200 μl media) were prepared from each mouse and cultured in triplicate wells with either (1) 1 μg/ml rBmVAL-1, (2) 1 μg/ml rBmALT-2, (3) 1 μg/ml rBmVAL-1+BmALT-2, (4) a nonspecific recombinant protein (1 μg/ml of *Schistosoma mansoni* G-binding protein) or (5) were left unstimulated in the media. All cells were incubated for 3 days at 37°C with 5% $CO_2$. After 3 days, $^3$H-Thymidine (0.5 lCi per well, Amersham Biosciences) was added to each well and further incubated. Cells were harvested 16 hours later and $^3$H-thymidine uptake was measured in a liquid scintillation counter and expressed as stimulation index (SI) = (counts per minute of stimulated cultures counts per minute of unstimulated cultures). Cell culture supernatants collected from the spleen cultures were assayed for IFN-γ, IL-4, IL-5 and IL-10 using an ELISA kit purchased from eBioscience Inc. (San Diego, CA).

*BmVAL-1 and BmALT-2 Specific IgG Antibodies in the Sera of Immunized Mice.* Titer of anti-BmVAL-1- and anti-BmALT-2-specific antibodies was determined in the sera of immunized mice using an ELISA (Veerapathran, et al. (2009) *supra*; Gnanasekar, et al. (2004) Infect. Immun. 72:4707-15). Pre-immune sera served as controls. HRP-conjugated goat anti-mouse IgG was used as the secondary antibody (Thermo Fisher Scientific) for mouse assays. OPD (Sigma) was used as the substrate and optical density (OD) was measured at 405 nm.

Anti-BmVAL-1- and anti-BmALT-2-specific IgG1, IgG2a, IgG2b, IgG3 and IgG4 antibodies were determined in the sera of mouse using a mouse antibody isotyping kit purchased from Thermo Fisher Scientific. All ELISAs were performed as per the manufacturer's recommendation and absorbance was read at 405 nm. Respective HRP-labeled goat anti-IgG isotype antibody was used as the secondary antibodies and color was developed using OPD substrate.

*Challenge Studies in Jirds.* Jirds were challenged with 100 *B. malayi* L3s and worm establishment was determined on day 95 after challenge according to established methods (Weil, et al. (1992) *supra*). Jirds are permissive hosts for *B. malayi* and the worms mature into adult males and females in about 75 days. Presence of mature worms in the control group of jirds was confirmed by demonstrating microfilariae in their blood on day 80 after challenge. Percent reduction in the worm establishment was calculated using the formula: average number of worms recovered from control worms - average number of worms recovered from vaccinated animals / average number of worms recovered from control animals x 100.

*Statistical Analysis.* Statistical analysis was performed using SIGMASTAT program (Jandel Scientific, San Rafel, California) and STATVIEW (SAS Institute, Cary, NC) software. Wilcoxon signed rank test was used to compare paired data; comparison between the groups was performed using the Mann-Whitney U test. p value of $p<0.05$ was considered statistically significant.

*EN individuals Carry High Titer of Antibodies Against BmVAL-1 and BmALT-2.* Significant anti-BmVAL-1 and anti-BmALT-2 IgG antibodies were present in the sera of EN subjects compared to MF subjects ($p < 0.01$) and CP subjects ($p < 0.005$). NEN subjects did not carry IgG antibodies against either of the antigens. Subsequent analysis of the IgG isotype of antibodies in the sera of EN subjects showed that anti-BmVAL-1 and anti-BmALT-2 antibodies were predominantly of IgG1 and IgG3 isotypes.

*High Titer of Antibody Responses in* the *Sera of Immunized Mice.* It has been shown that mice vaccinated with *B. malayi* antigens elicit significant host protective IgG antibodies. Therefore, IgG antibody titers in the sera of immunized mice were determined. Monovalent immunization with *BmVal-1* and monovalent immunization with *BmAlt-2* both elicited significant ($p< 0.005$) titers of anti-BmVAL-1 and anti-BmALT-2 IgG antibodies in the sera of mice. Compared to controls, the prime boost immunized group gave the maximum titer of antibodies followed by protein immunized and DNA immunized groups. Immunization with the multivalent vaccine formulation *(BmVAL-1* + *BmALT-2)* also elicited significant IgG antibody titers against both rBmVAL-1 and rBmALT-2 and the titers were comparable, indicating that the antigens do not interfere with each other or compete for dominance. An interesting finding was that the multivalent vaccine elicited significantly higher ($p< 0.001$) titer of IgG antibodies in mice compared to any of the monovalent vaccines. These finding indicated that the two antigens in the multivalent formulation synergistically increased the vaccine-induced antibody responses.

Overall, protein vaccination elicited higher titer of IgG antibodies compared to DNA vaccines, indicating that protein vaccinations were highly immunogenic. Another observation was that a heterologous prime boost approach gave a higher seroconversion than homologous prime boost approach. Thus, overall heterologous prime boost approach appeared to stimulate the highest titer of antibodies.

IgG antibody subset analysis showed that BmVAL-1 vaccination elicited primarily IgG1 and IgG2a isotype of antibodies, whereas, BmALT-2 vaccination induced IgG1, IgG2a and IgG3 isotype of antigen-specific antibody responses. Antigen-specific IgG4 antibody responses were not evident. The prime boost approach significantly amplified the IgG isotype responses. Following multivalent vaccination regimen IgG1, IgG2a and IgG3 subset of antigen specific antibodies were present in the sera of mouse.

*Antigen-Specific Responses in the Spleen of Mice.* Spleen cells from immunized mice stimulated with either rBmVAL-1 or rBmALT-2 proliferated significantly (SI $10.8 \pm 1.1$ and SI $14.6 \pm 1.2$, respectively) compared to the media control (SI $2.1 \pm 0.9$). Spleen cells from mice immunized with the multivalent construct responded to both rBmVAL-1 (SI $18.9 \pm 2.6$) and rBmALT-2 (SI $23.5 \pm 3.1$), indicating that a strong recall cellular response was generated to both BmVAL-1 and BmALT-2 following vaccination with the multivalent construct.

*Cytokine Analysis from Proliferated* Culture *Supernatants.* To identify the cytokine profile of the antigen-responding cells, the culture supernatant of mouse spleen cells stimulated with respective antigen (rBmVAL-1 or rBmALT-2) was collected and the level of IFN-$\gamma$, IL-4, IL-5 and IL-10 was measured. These results showed that significant levels of IL-5 and IFN-$\gamma$ were secreted by the spleen cells in response to rBmVAL-1. Spleen cells stimulated with rBmALT-2 predominantly secreted IL-4 and IL-5.

*Multivalent Vaccine Induces Significant Protection in Mice and Jirds.* The results herein indicated that significant IgG antibodies were elicited following vaccination with monovalent and multivalent vaccine preparations. To test if the immune responses elicited following vaccination were protective, vaccinated animals were challenged with live, third stage infective larvae (L3) of *B. malayi.* Since the parasites do not reach to maturity in mice, a standard micropore chamber challenge method was used (Gnanasekar, et al. (2004) *supra*). These studies showed that 39% to 74% protection was achieved in mice following immunization with monovalent vaccine (Table 9).

TABLE 9

| Vaccination Group | Mean±SD Live L3s | Percent Protection |
|---|---|---|
| *pVAXBmVAL-1* DNA monovalent homologous | 12.2±4.5 | 39.0+1.7%** |
| rBmVAL-1 protein monovalent homologous | 10.4±3.1 | 48.0±2.1%* |
| *pVAXBmVAL-1* DNA plus rBmVAL-1 monovalent heterologous | 9.2±2.2 | 54.0±3.1%* |
| *pVAXBmALT-2* DNA monovalent homologous | 9.8±2.1 | 51.0±2.5%* |
| rBmALT-2 protein monovalent homologous | 7.0±1.1 | 65.0±4.2%* |
| *pVAXBmALT-2* DNA plus rBmALT-2 monovalent heterologous | 5.1±0.5 | 74.5±3.1%* |
| *pVAXBmVAL-1/ALT-2* DNA multivalent homologous | 8.6±0.1 | 57.0±2.2%* |
| rBmVAL-1/rBmALT-2 protein multivalent homologous | 5.2±1.1 | 74.0±3.3%* |
| *pVAXBmVAL-1/BmALT-2* DNA plus rBmVAL-1/rBmALT-2 multivalent heterologous | 4.4±0.4 | 82.0±2.2%* |

(continued)

| Vaccination Group | Mean±SD Live L3s | Percent Protection |
|---|---|---|
| *pVAX* + Alum control | 20±0 | 0% |
| Significance, * p<0.01, **p<0.05 compared to control. | | |

Protein vaccination gave better results than DNA vaccination. The prime boost regimen gave the best results overall. Vaccination with BmALT-2 gave higher percent of protection compared to BmVAL-1. Similarly, multivalent vaccination regimen gave the 57% to 82% protection compared to the monovalent vaccination regimen. These finding indicated that BmVAL-1 and BmALT-2 synergistically enhance the protective immune responses in vaccinated animals when given as a multivalent vaccine.

Analysis of the thick blood smear prepared from the control group of jirds on day 80 after challenge showed that all five jirds were positive for microfilaria, whereas, microfilaria were not detected in the peripheral blood of vaccinated jirds. Fifteen (15) days later the animals were sacrificed and the male and female worms in the peritoneal, pelvic and pleural cavities were counted and the results between controls and vaccinated groups were compared (Table 10). Findings from vaccination of jirds also confirmed that the multivalent prime boost regimen gave the highest rate of protection. No female worms were recovered from the multivalent vaccinated animals.

TABLE 10

| Vaccination Group | Percent Production |
|---|---|
| *pVAXBmVAL-1* DNA monovalent homologous | 50±3.7% |
| rBmVAL-1 protein monovalent homologous | 40.0±3.1% |
| *pVAXBmVAL-1* DNA plus rBmVAL-1 monovalent heterologous | 52.4±2.5% |
| *pVAXBmALT-2* DNA monovalent homologous | 58.3±2.1% |
| rBmALT-2 protein monovalent homologous | 72.0±5.5% |
| *pVAXBmALT-2* DNA plus rBmALT-2 monovalent heterologous | 78.5±3.2% |
| *pVAXBmVAL-1/ALT-2* DNA multivalent homologous | 77.1±2.0% |
| rBmVAL-1/rBmALT-2 protein multivalent homologous | 79.9±3.5% |
| *pVAXBmVAL-1/BmALT-2* DNA plus rBmVAL-1/rBmALT-2 multivalent heterologous | 85.0±1.4% |
| *pVAX* + Alum control | 0 |
| Significance, p<0.01 compared to control. | |

**Example 4: WbHSP+WbALT-2+WbTSP Multivalent Vaccine**

*Parasites. Brugia malayi* L3s were obtained from the NIAID/NIH Filariasis Research Reagent Resource Center (FR3) at the University of Georgia, Athens, GA.

*Construction of pVAX Wbhsp+Wbalt2+Wbtsp DNA vaccine.* The codon-optimized DNA sequence coding for *Bmhsp* was amplified with the forward primer 5'-CGC GGA TCC ACC GTG ATC CAT TGT CG-3' (SEQ ID NO:25) containing *Bam*HI restriction site and the reverse primer 5'-AAC TGC AGC TGT TTT CCA TTT CCA TTC-3' (SEQ ID NO:26) containing PstI restriction site without the stop codon and cloned into pVAX vector. The resulting plasmid was designated as pVAX Wbhsp*. Codon-optimized Wbalt-2 gene was amplified with the forward primer 5'-AAC TGC AGA TGG GTA ACA AGC TCC TCA TCG-3' (SEQ ID NO:27) and the reverse primer without the stop codon 5'-CGC GAA TTC GGC GCA CTG CCA ACC TGC-3' (SEQ ID NO:28). Underlined sequences indicate PstI and EcoRI restriction sites in the forward and reverse primers, respectively. The amplified Wbalt-2 DNA insert was then subcloned into pVAX Wbhsp* plasmid at the PstI and EcoRI restriction sites, resulting in pVAX Wbhsp+Wbalt-2* plasmid. To clone the final product of pVAXWbhsp+Wbalt-2+Wbtsp plasmid, the gene sequence encoding Wbtsp ECL domain alone was amplified with the forward primer 5'-CGC GAA TTC ACC ATG GTC CTG GAG-3' (SEQ ID NO:29) containing EcoRI restriction site and the reverse primer with stop codon 5'-GCT CTA GAT CAG TCC TTC TGG CTA G-3' (SEQ ID NO:30) containing *Xba*I restriction site and cloned into pVAX Wbhsp+Wbalt-2* plasmid. Bivalent constructs of HSP+TSP, TSP+ALT and HSP+ALT were also constructed with their respective primers.

*Construction of pRSETA Wbhsp+Wbalt2+Wbtsp, a Multivalent Fusion Protein.* pRSETA WbHSP+WbALT-2+WbTSP was constructed in the same manner as above. The primer sequences of HSP, ALT-2 and TSP were as follows. WbHSP, forward primer, 5'-CGG GAT CCA TGG AAG AAA AGG TAG TG-3' (SEQ ID NO:31) containing BamHI and reverse primer, 5'-CCC TCG AGT GCT TTC TTT TTG GCA GC-3' (SEQ ID NO:32) containing *Xho*I. WbALT-2, forward primer, 5'-CCC TCG AG A TGA ATA AAC TTT TAA TAG CAT-3' (SEQ ID NO:33) containing *Xho*I and reverse primer, 5'-GGG TAC CCG CGC ATT GCC AAC CC-3' (SEQ ID NO:34) containing *Kpn*I. WbTSP, forward primer, 5'-GGG GTA CCC CGG CAA GGA TCA ATT TAA AA-3' (SEQ ID NO:35) containing *Kpn*I and reverse primer, 5'-CGG AAT TCT CAA TCT TTT TGA GAT GAA T-3' (SEQ ID NO:36) containing EcoRI. Similarly bivalent constructs (HA, HT and TA) were also cloned individually into a pRSETA vector.

*Immunization of Animals.* Six-week-old Balb/C mice were immunized with 100 $\mu$g of DNA intradermally (i.d.) as DNA vaccine or with 15 $\mu$g of recombinant protein subcutaneously (s.c.) as protein vaccine or with two doses of DNA and two doses of protein as prime-boost vaccine. Mice were randomly divided into 15 groups with 5 mice per group. Animals from groups 1-3 were immunized with HSP+ALT-2 (HA). Groups 4-6 were immunized with HSP+TSP (HT), and 7-9 were immunized with TSP+ALT-2 (TA). Mice from groups 10-12 were immunized with the multivalent vaccine HSP+ALT-2+TSP (HAT). Control group of animals received pVAX vector and/or alum (Infectious Disease Research Institute (IDRI)). This experiment was repeated twice with all the groups.

*Analysis of Antibody Response in Immunized Animals.* IgG antibody levels in the sera of immunized and control groups of animals against all the three proteins were determined using an indirect ELISA (Anandharaman, et al. (2009) *supra).* Briefly, wells of a 96-well microtiter plate were coated with recombinant proteins (rHSP, rALT-2 or rTSP; 1 $\mu$g/ml) in carbonate buffer, pH 9.6, overnight at 4°C and blocked with 3% BSA for 1 hour at 37°C. Sera samples were added to the wells and the plates were incubated overnight at 4°C. After washing, HRP-labeled mouse anti-human IgG was added (1:5000) and incubated further for 1 hour at 37°C. The color was developed with OPD (o-phenylene diamine) substrate (Sigma Aldrich, USA). Absorbance was measured at 450 nm in a microplate reader (BIO-RAD, Hercules, CA).

Immunoblot analysis was also performed with the immunized mice sera. Sera samples from the mice immunized with the recombinant proteins (rHAT, rALT2, rTSP or rWbHAT) were used for the immunoblot study. The color of the blot was developed with the diaminobenzidine (DAB) substrate.

*ADCC Assay.* To evaluate the protection efficacy of the antigen combinations, *in vitro* ADCC was performed with the sera from the mice immunized with bivalent and trivalent vaccine constructs. The *in vitro* ADCC assay was performed according to known methods (Chandrasekhar, et al. (1990) *supra*). Briefly, Peritoneal Exudates Cells (PEC) were collected from normal Balb/c mice by washing the peritoneal cavity with sterile RPMI 1640 media. The cells were washed and suspended in RPMI 1640 medium supplemented with 10% Fetal Calf Serum (FCS). Ten $L_3$ of *B. malayi* were added to 2 x $10^5$ peritoneal exudates cells (PEC)/well in 96-well culture plates (Thermo Fisher Scientifics, USA.), 50 $\mu$l of immunized mice sera and 50 $\mu$l of RPMI 1640 media were added to the wells in triplicates and incubated for 48 hours in 5% $CO_2$ at 37°c. Larval viability was determined microscopically after 48 hours of incubation. Larvae that were limpid, damaged and with the clumps of cells adhered to it were counted as dead. ADCC was estimated as the percent larval death calculated using the formula: Number of Dead larvae ÷ Total number of larvae x 100.

*Depletion of IgG Antibodies from the Sera Samples.* Sera from mice immunized with multivalent vaccine was depleted of recombinant antigen specific IgG antibodies using cobalt IMAC resin coupled with his-tagged recombinant antigens (Anandharaman, et al (2009) *supra).* Briefly, 1 mg of his-tagged recombinant protein (rHSP) was coupled to 2 ml bed volume of IMAC resin for 2 hours at 37°C. The cobalt column was washed with ten bed volumes of PBS (pH.8) and incubated overnight at 4°C with 200 $\mu$l of pooled sera from the mice immunized with multivalent vaccine. Supernatant containing the depleted sera was collected by centrifugation. Anti-HSP-depleted serum was incubated overnight at 4°C in rALT-2-coupled column. The supernatant containing anti-HSP- and anti-ALT-2-depleted serum was collected and incubated in rTSP-coupled column. Anti-HSP-, anti-ALT-2-, and anti-TSP-depleted serum was collected and used. Depletion of IgG antibodies against specific antigens was confirmed by ELISA as described above. Antibody-depleted sera were then used in an ADCC assay.

*Analysis of in situ Cytotoxicity Against* L3 *Larvae in Immunized Mice (Micropore Chamber Technique).* The protective efficacy of vaccination was analyzed by challenging the immunized animals with infective L3 using micropore chamber method (Abraham, et al. (1989) *supra).* Micropore chambers were assembled using 14 x 2 mm PLEXI rings and 5.0 $\mu$m nucleopore polycarbonate membranes (Millipore Corporations, Bedford, MA). After 48 hours of implantation, animals were sacrificed and the chambers were recovered from peritoneal cavity. Contents of each chamber were examined microscopically for cell adherence and death of infective L3. The parasite was considered dead if it was not motile and limpid, and had several adherent cells on the surface. The percentage protection was calculated using the formula: number of dead parasites ÷ number of recovered parasites x 100. This experiment was repeated twice with five animals in each group.

*Splenocyte Proliferation.* Vaccinated and control mice were sacrificed on day 60 and the spleens were removed aseptically. Single-cell suspensions were prepared in RPMI 1640 medium supplemented with 10% heat-inactivated FCS, passed through a NYLON mesh (BD Biosciences, Bedford, USA). After determining the viability of cells using trypan blue dye exclusion, approximately 2 x $10^6$ cells per well in triplicates were plated in 96-well culture plates (ThermoFisher, USA). The splenocytes were stimulated with 1μg/100μl/well of recombinant proteins (rHSP, rALT-2 or rTSP) or ConA or with medium alone (Unstimulated) for 72 hours at 37°C in the atmosphere of 5% $CO_2$. Cell proliferation was determined using cell counting kit (CCK-8) purchased from Dojindo Molecular Technologies, Inc. (Gaithersburg, MD). Stimulation index of spleen cell proliferation was calculated using the formula: Absorbance of stimulated cells ÷ Absorbance of unstimulated cells. All cultures were taken in triplicates and the results expressed as mean S.I.±SEM.

*Real Time-PCR (RT-PCR)*. Cytokine levels in the mRNA of the spleen cell pellets were analyzed by real time-PCR. The spleen cells of vaccinated and control group mice were cultured as above at a concentration of $2x10^6$ cells/100μl/well in 96-well plates and stimulated with recombinant antigens (1 μg/ml). After 72 hours, cells were centrifuged (1000 rpm for 5 minutes) and total RNA was extracted from the cell pellets using TRIZOL reagent (Invitrogen) as per description of the manufacturer. Followed by RNA extraction, first-strand cDNA was synthesized by RT2 First Strand Kit (SuperArray Bioscience Corporation, Frederick, MD). PCR array analysis was performed according to the manufacturer protocol with the RT2 Real-Time TM SYBR Green PCR Master Mix. Aliquots from this mix were added to a 96-well plate, where each well contained predispensed gene-specific primer sets. Relative quantification of the genes of interest that expressed was measured in an Applied BioSystem 7300 real-time PCR machine (Applied BioSystems, Foster City, CA). Cycling parameters were as follows: 95°C for 10 minutes for activation of HOTSTART DNA polymerase, followed by 40 cycles of denaturation at 95°C for 15 seconds and primer extension at 60°C for 1 minute. RT-PCR data array set was generated and analyzed using SABiosciences web-based data analysis system. Results were expressed in terms of fold change of immunized mice compared to control mice by normalizing the expression of housekeeping genes.

*Cytokine Assay.* Splenocyte cell culture supernatants were collected after 72 hours incubation stimulated with recombinant antigens (1 μg/ml) or with medium alone. Secreted levels of IL-4 and IFN-γ cytokines in the culture supernatants were determined using a sandwich ELISA kit purchased from Thermo Scientifics, USA. All concentrations were derived from standard curves and data expressed in pg/ml.

*Construction of cHAT Plasmid and Expression of Fusion Proteins.* Since the N-terminal region of HSP is involved in IL-10 binding, this region was deleted and cHAT recombinant protein was prepared as 37 KDa His-tagged protein.

Construction of Recombinant Plasmids and Expression of Fusion Proteins. The full-length *hsp, alt-2* and *tsp* genes of *B. malayi* L3 stage were constructed with the expected size (850 bp). These fragments were further directionally cloned into the expression vectors pVAX1 and pRSETA with the specified restriction enzyme cutting sites. Results of the DNA sequence analysis confirmed gene insertion direction. rWbHAT was expressed as a 45 KDa His-tagged fusion protein, which was purified and analyzed in SDS-PAGE. The results indicated that the fusion protein was pure without any contaminating proteins. The presence of antibodies against all the three antigens was confirmed by immunoblot analysis.

*Antibody Titer in* the *Immunized Mice Sera.* The mean peak antibody titer of the sera samples from the mice immunized with prime-boost or protein vaccine was significantly higher (p<0.001) compared to the DNA group. Sera collected from rWbHAT-immunized animals showed the maximum titer of 30,000 against rALT-2 antigen, while the antibody titer against rHSP or rTSP antigen was in the range of 18,000-20,000. Similarly, the mice immunized with the bivalent vaccine showed the maximum titer of 30,000 against ALT-2 antigen while anti-HSP and anti-TSP antibodies were in the range of 8,000-15,000.

*Antibody-Dependent Cell-Mediated Cytotoxicity.* Antibody-mediated adherence and cytotoxicity of immune cells to *B. malayi* L3 larvae was observed after 48 hours of incubation of parasites, with the sera and normal immune cells. ADCC showed maximum cytotoxicity of approximately 90% (p<0.001) in the sera of mice immunized with rWbHAT or rWbHA vaccine constructs (Table 11). Bivalent vaccine constructs of rWbHT and rWbTA also gave better protection of 82% and 87%, respectively, which was significant compared to monovalent-vaccinated and control animals (p<0.001). To evaluate the protection mediated by the antibodies generated against HSP, ALT and TSP antigens, IgG antibodies were depleted from the immunized sera and used in ADCC. Depleted antibodies showed only 6% protection against L3.

TABLE 11

| Groups | % Cytotoxicity |
|---|---|
| H+A | 90±2.4* |
| H+T | 82.30±12.9* |
| T+A | 87.06±9.8* |
| H+A+T | 88.69±7.5* |
| anti-HSP + anti-ALT + anti-TSP antibodies depleted from HAT immunized sera | 5.55+1.5 |
| Values represent mean±SD of three wells. *Significant larval death (P<0.001) compared to other mice groups. | |

*In situ Protection Study.* Two weeks after the final immunization, the ability of the vaccine candidates, to kill the filarial parasites in the immunized animals was evaluated by in situ micropore chamber studies. The data was combined from the two similar experiments and represented as mean count ± SEM. The analysis of percentage reduction in worm burden compared with control showed that multivalent vaccine (HAT) conferred the maximum protection of 100% and 94% for protein and prime-boost vaccine, which was very significant protection (Table 12) (P<0.0001) compared to control groups (5%). Interestingly, the percentage worm reduction of bivalent vaccines HA, TA and HT were 90%, 80% and 82%, respectively, which was also significantly high compared to the control. In the entire bivalent vaccine group, prime-boost vaccination was more protective compared to DNA and protein vaccination.

TABLE 12

| Trial 1 DNA Vaccine | | Trial 2 Protein Vaccine | | Trial 3 Prime-Boost Vaccine | |
|---|---|---|---|---|---|
| Group | % Cytotoxicity | Group | % Cytotoxicity | Group | % Cytotoxicity |
| pVAX | 5 ± 4.23 | Alum | 3 ± 4.23 | pVAX+Alum | 5.9 ± 4.23 |
| H+A | 81 + 11.23* | H+A | 78 + 11.23* | H+A | 90 + 11.23* |
| H+T | 72 ± 12.03* | H+T | 69 ± 12.03* | H+T | 80 + 12.03* |
| T+A | 74 ± 11.21* | T+A | 66 ± 11.21* | T+A | 82 ± 11.21* |
| H+A+T | 91 ± 11.92* | H+A+T | 100 ± 0* | H+A+T | 94 ± 11.92* |
| Values are mean ± SD. N=5. Data is from one of two similar experiments showing comparable results. *Significant larval death (P<0.001) compared to other mice groups. | | | | | |

*Splenocyte Proliferation.* Spleen cells isolated from vaccinated and control animals were stimulated *in vitro* individually with rHSP, rALT-2 or rTSP to analyze the protein-specific T-cell proliferation in vaccinated animals. Mice immunized with the prime-boost regimen in all the vaccine combinations and HAT as protein vaccine gave the highest protection. Hence the splenocytes were collected only from these animals analyzed for the immune response. Splenocytes from bivalent- and trivalent-vaccinated animals stimulated with respective recombinant proteins showed significantly high (P<0.001) proliferation (mean S.I. = 4.25-5.8) when compared to monovalent and unstimulated controls. The proliferation index of spleen cells immunized with the monovalent construct showed significant proliferation. The stimulation of cells was comparable to the positive controls.

*RT-PCR Array.* To determine the cellular immune responses to multivalent constructs in the vaccinated mice, spleen cells collected from vaccinated and control mice were cultured in the presence of respective recombinant proteins and their proliferative responses and cytokine profiles were evaluated. Since the spleen cells from vaccinated animals were proliferating significantly to recall response, levels of cytokine mRNA were measured. An RT-PCR cytokine gene array was performed on mRNA collected from the spleen cells stimulated with recombinant proteins. These results showed that both Th1 (IFN-$\gamma$, IL-2) and Th2 (IL-4) cytokine genes were significantly increased in vaccinated animals.

*Cytokine Levels.* After identifying the presence of IFN-$\gamma$ and IL-4 cytokine expression in the mRNA isolated from the vaccinated spleen cells, the secretion of same cytokines in the supernatant was investigated. The data were normalized with the unstimulated controls. Interestingly, the cytokine profiles observed in the supernatant exhibited significantly higher levels of IFN-$\gamma$ showing a Th1-biased immune response. These results demonstrated that recombinant proteins stimulated the production of IFN-$\gamma$ and induced a Th1-mediated protective response.

**Example 5: Analysis of cHAT Vaccine in Various Adjuvant Formulations**

*Preparation of cHAT.* Previous studies showed that the N-terminal sequence of BmHSP12.6 can bind to human IL-10 receptor and trigger IL-10 mediated responses (Gnanasekar, et al. (2008) Mol. Biochem. Parasitol. 159(2):98-103. Since IL-10 is an immunosuppressive agent, the IL-10 receptor binding sequences were deleted from HSP. The truncated sequence was referred to as cHSP. The cHSP was then used to replace the HSP gene and HSP protein in the multivalent HAT hybrid vaccine. Thus, the resulting new vaccine was called cHAT.

*Protection Studies Using cHAT-Fusion Protein Vaccine in Mice.* Mice were immunized with four doses of cHAT fusion protein at two week intervals. One month after the final immunization, the ability of the vaccine candidates to kill the filarial parasites was evaluated by *in situ* micropore chamber studies. Results showed that when mice were immunized with cHAT fusion protein with alum as the adjuvant, the vaccine conferred 81% protection (Table 13) (P < 0.0001) compared to control groups (2%) that received only phosphate-buffered saline (PBS) and alum. Different adjuvants were then tested to see if changing the adjuvant would improve the protection ability of cHAT. Two additional adjuvants were tested: alum containing a TLR4 agonist (purchased from Infectious Disease Research Institute, Seattle, Washington) and ALHYDROGEL (purchased from Sigma, St. Louis, MO). cHAT with no adjuvants remained as a control. Results from these studies (Table 13) showed that 78% protection was achieved with alum plus TLR4 agonist and cHAT given in ALHYDROGEL adjuvant gave 70% protection. An interesting finding in these studies was that cHAT without any adjuvant also gave 72% protection indicating that the cHAT fusion protein vaccine could be administered without any adjuvant and still obtain significant protection.

TABLE 13

| Group | % Larval Death (Mean±SD) |
|---|---|
| cHAT + Alum | 81 ± 7.8 |
| PBS + Alum Control | 1.7 + 1.3 |
| cHAT + Alum with TLR4 agonist | 78 ± 8.4 |
| cHAT + ALHYDROGEL | 70 ± 13 |
| cHAT With No adjuvant | 72 ± 12 |
| Values are mean ± SD. N=5. Data is from one of two similar experiments showing comparable results. *Significant larval death (P<0.001) compared to other mice groups. | |

**Example 6: Homologues of HSP, ALT-2 and TSP**

Homologues of the vaccine antigens, HSP, ALT-2 and Tetraspanin are present in *O. volvulus* and L. *loa.* Comparison of the nucleotide sequence of HSP, ALT-2 and Tetraspanin from *O. volvulus* and L. *loa* show that there is significant sequence homology (>90%) between the proteins from all filarial parasites. These findings indicate that the cHAT fusion protein vaccine developed in Example 5 can be used as a vaccine against *O. volvulus* and L. *loa.*

As an example, *O. volvulus* tetraspanin was cloned from *O. volvulus* L3 cDNA library and recombinant proteins were prepared. Sera sample from mice vaccinated with cHAT vaccine that gave the 81% protection in Table 13 was used to probe the recombinant *O. volvulus* tetraspanin after separating the protein in a 12% SDS-PAGE gel. *B. malayi* tetraspanin was used as a positive control. Results showed that the sera sample significantly reacted with *O. volvulus* tetraspanin (Figure 4) thereby indicating that the cHAT vaccine developed in Example 5 is of use as a vaccine against *O. volvulus.*

## Claims

1. A fusion protein comprising three or more covalently attached isolated antigens from one or more filarial nematodes, wherein the antigens comprise Abundant Larval Transcript (ALT-2), Tetraspanin, and Small heat shock protein (HSP) 12.6, or fragments thereof, wherein the fragment is selected from the group of SEQ ID NO:39, SEQ ID NO:63, and SEQ ID NO:64.

2. The fusion protein of claim 1, wherein the filarial nematodes are selected from the group of *Brugia malayi, Wuchereria bancrofti Onchocerca volvulus, Loa loa* and *Brugia timori.*

3. An isolated nucleic acid encoding the fusion protein of any preceding claim.

4. A recombinant vector comprising a nucleic acid according to Claim 3.

5. The recombinant vector of claim 4, wherein the nucleic acid is selected from the group of SEQ ID NO:65, SEQ ID NO:66, SEQ ID NO:67, SEQ ID NO:68, and SEQ ID NO:69.

6. A recombinant host cell comprising the recombinant vector of claim 4 or 5.

7. The fusion protein of claim 1 for use in immunizing an animal against filariasis, the use comprising administering to an animal in need thereof the fusion protein of claim 1.

8. A multivalent vaccine comprising at least one of the fusion proteins of any of Claims 1-2, or the nucleic acid of Claim 3.

9. The multivalent vaccine of Claim 8 for use in immunizing an animal against filariasis, the use comprising administering to an animal in need thereof the multivalent vaccine of claim 8.

## Patentansprüche

1. Fusionsprotein, umfassend drei oder mehr kovalent angehängte isolierte Antigene von einem oder mehreren Fadenwürmern, wobei die Antigene Abundant Larval Transcript (ALT-2), Tetraspanin und Small Heat Shock Protein (HSP) 12.6 oder Fragmente davon umfassen, wobei das Fragment aus der Gruppe der SEQ ID NO:39, SEQ ID NO:63 und SEQ ID NO:64 ausgewählt ist.

2. Fusionsprotein nach Anspruch 1, wobei die Fadenwürmer aus der Gruppe der *Brugia malayi, Wuchereria bancrofti, Onchocerca volvulus, Loa loa* und *Brugia timori* ausgewählt sind.

3. Isolierte Nukleinsäure, die das Fusionsprotein nach einem vorhergehenden Anspruch codiert.

4. Rekombinanter Vektor, umfassend eine Nukleinsäure nach Anspruch 3.

5. Rekombinanter Vektor nach Anspruch 4, wobei die Nukleinsäure aus der Gruppe der SEQ ID NO:65, SEQ ID NO:66, SEQ ID NO:67, SEQ ID NO:68 und SEQ ID NO:69 ausgewählt ist.

6. Rekombinante Wirtszelle, umfassend den rekombinanten Vektor nach Anspruch 4 oder 5.

7. Fusionsprotein nach Anspruch 1 zur Verwendung beim Immunisieren eines Tieres gegen Filariose, wobei die Verwendung das Verabreichen des Fusionsproteins nach Anspruch 1 an ein Tier, das dessen bedarf, umfasst.

8. Multivalenter Impfstoff, umfassend zumindest eines der Fusionsproteine nach einem der Ansprüche 1-2 oder die Nukleinsäure nach Anspruch 3.

9. Multivalenter Impfstoff nach Anspruch 8 zur Verwendung beim Immunisieren eines Tieres gegen Filariose, wobei die Verwendung das Verabreichen des multivalenten Impfstoffes nach Anspruch 8 an ein Tier, das dessen bedarf, umfasst.

**Revendications**

1. Protéine de fusion comprenant trois antigènes isolés liés de manière covalente ou plus d'un ou plusieurs nématodes filariens, dans laquelle les antigènes comprennent un ALT-2 (abundant larval transcript), la tétraspanine, et une petite HSP (small heat shock protein) 12.6, ou leurs fragments, dans laquelle le fragment est sélectionné dans le groupe consistant en SEQ ID NO : 39, SEQ ID NO : 63, et SEQ ID NO : 64.

2. Protéine de fusion selon la revendication 1, dans laquelle les nématodes filariens sont sélectionnés dans le groupe de *Brugia malayi, Wuchereria bancrofti, Onchocerca volvulus, Loa loa* et *Brugia timori.*

3. Acide nucléique isolé codant pour la protéine de fusion selon l'une quelconque des revendications précédentes.

4. Vecteur recombinant comprenant un acide nucléique selon la revendication 3.

5. Vecteur recombinant selon la revendication 4, dans lequel l'acide nucléique est sélectionné dans le groupe de SEQ ID NO : 65, SEQ ID NO : 66, SEQ ID NO : 67, SEQ ID NO : 68, et SEQ ID NO : 69.

6. Cellule hôte recombinante comprenant le vecteur recombinant selon la revendication 4 ou 5.

7. Protéine de fusion selon la revendication 1 pour son utilisation dans l'immunisation d'un animal contre la filariose, l'utilisation comprenant l'administration à un animal le nécessitant de la protéine de fusion selon la revendication 1.

8. Vaccin multivalent comprenant au moins l'une des protéines de fusion selon l'une quelconque des revendications 1 et 2, ou l'acide nucléique selon la revendication 3.

9. Vaccin multivalent selon la revendication 8 pour son utilisation dans l'immunisation d'un animal contre la filariose, l'utilisation comprenant l'administration à un animal le nécessitant du vaccin multivalent selon la revendication 8.

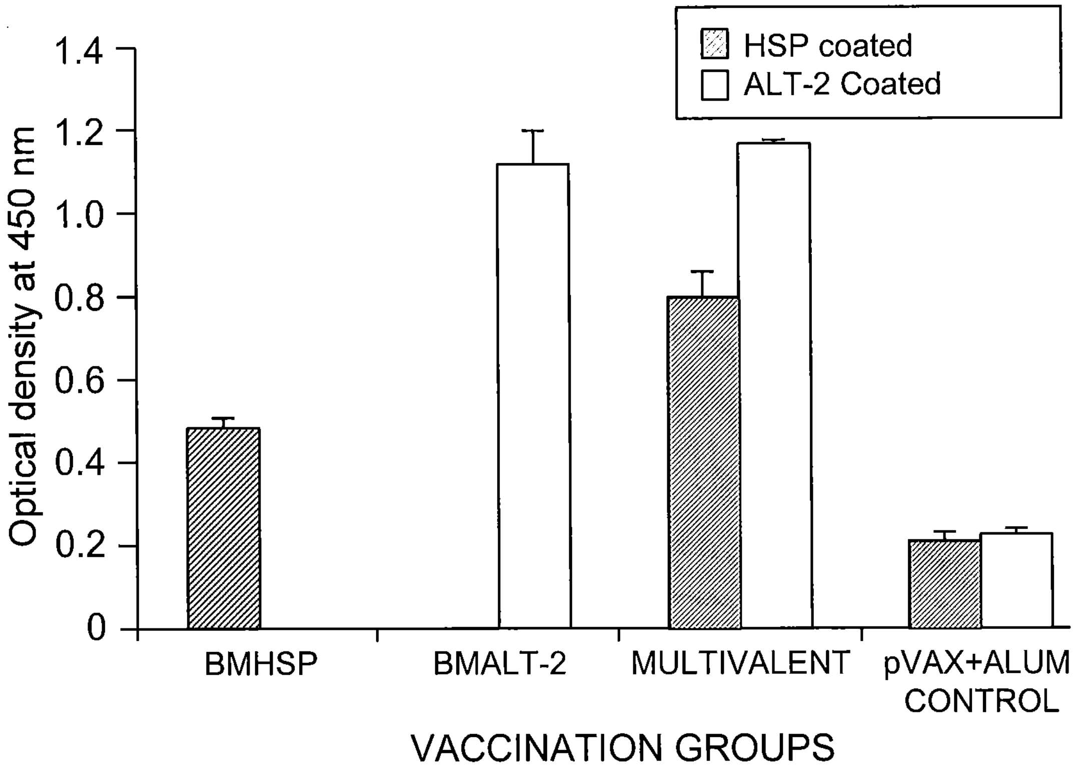


*FIG. 1*

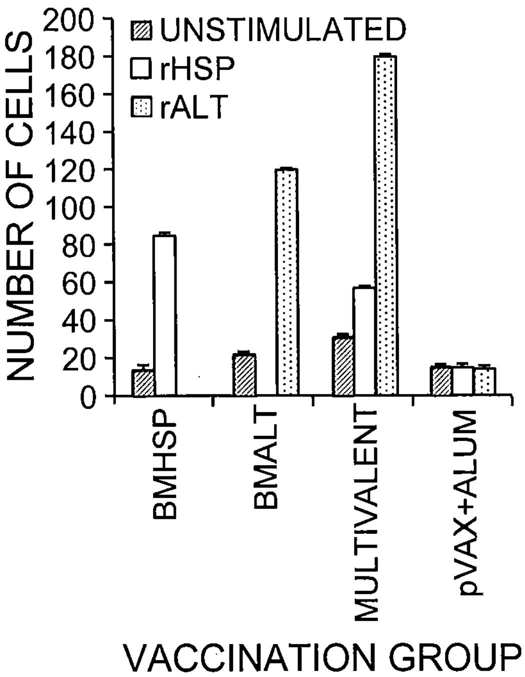


*FIG. 2A*

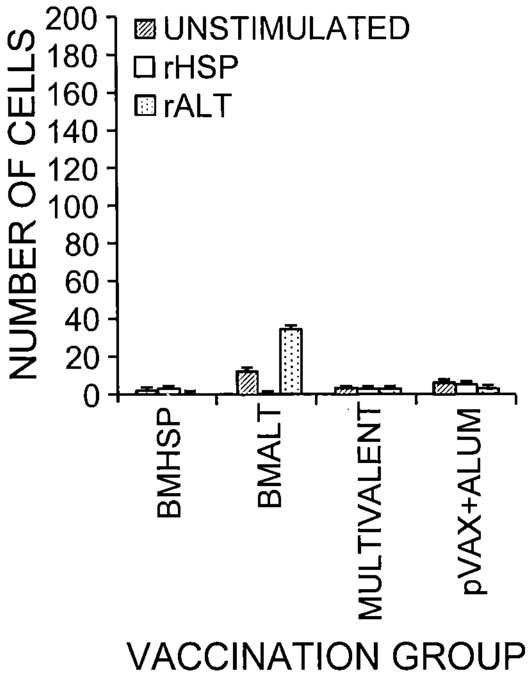


*FIG. 2B*

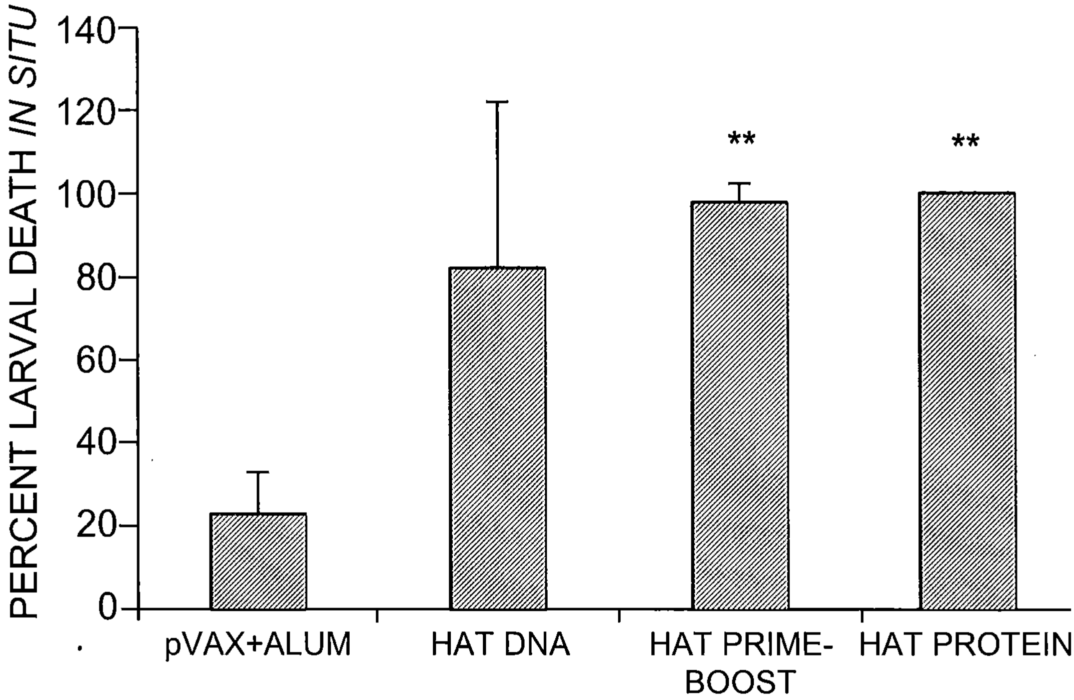
PERCENT LARVAL DEATH *IN SITU*
140
120
100
80
60
40
20
0
**
**
pVAX+ALUM
HAT DNA
HAT PRIME-BOOST
HAT PROTEIN

*FIG. 3*

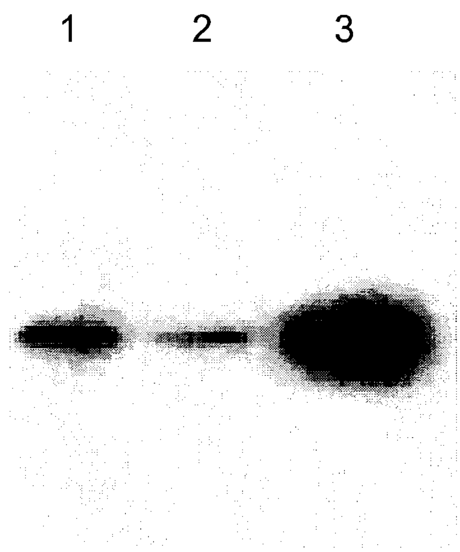
1
2
3

*FIG. 4*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61413681
- US 61449954
- US 61522079
- WO 9417813 A
- US 61413681 B
- US 61449954 B
- US 61522079 B
- US 2011059501 W


### Non-patent literature cited in the description


- *World Health Organ. Tech. Rep. Ser.,* 1992, vol. 821, 1-71
- **HOTEZ.** *Clin. Pharmacol. Ther.,* 2009, vol. 85 (6), 659-64
- **BABU.** *Trans. R. Soc. Trop. Med. Hyg.,* 2008, vol. 102 (12), 1207-13
- **EL-SETOUHY et al.** *Am. J. Trop. Med. Hyg.,* 2007, vol. 77 (6), 1069-73
- **HORTON.** *Ann. Trop. Med. Parasitol.,* 2009, vol. 103 (1), 33-40
- **SCHWAB et al.** *Parasitology,* 2007, vol. 134, 1025-40
- **BOTTAZZI et al.** *Expert Rev. Vaccines,* 2006, vol. 5 (2), 189-98
- **CHENTHAMARAKSHAN et al.** *Parasite Immunol.,* 1995, vol. 17 (6), 277-85
- **DISSANAYAKE et al.** *Am. J. Trop. Med. Hyg.,* 1995, vol. 53 (3), 289-94
- **LI et al.** *J. Immunol.,* 1993, vol. 150 (5), 1881-5
- **MAIZELS et al.** *Int. J. Parasitol.,* 2001, vol. 31 (9), 889-98
- **THIRUGNANAM et al.** *Exp. Parasitol.,* 2007, vol. 116 (4), 483-91
- **VEERAPATHRAN et al.** *PLoS Negl. Trop. Dis.,* 2009, vol. 3 (6), e457
- **GNANASEKAR et al.** *Infect. Immun.,* 2004, vol. 72 (8), 4707-15
- **COOKSON et al.** *Proc. Natl. Acad. Sci. USA,* 1992, vol. 89, 5837-5841
- **MAIZELS et al.** *Parasitology,* 1983, vol. 87, 249-263
- **MAIZELS et al.** *Clin, Exp. Immunol.,* 1983, vol. 51, 269-277
- **NOORDIN et al.** *Filaria J.,* 2004, vol. 3, 10
- **KRON et al.** *FEBS Lett.,* 1995, vol. 374, 122-4
- **SELKIRK et al.** *J. Immunol.,* 1989, vol. 143, 299-308
- **LI et al.** *Mol. Biochem. Parasitol.,* 1991, vol. 49, 315-23
- **WOLFF et al.** *Science,* 1990, vol. 247, 1465-1468
- **SALI ; BLUNDELL.** *J. Mol. Biol.,* 1993, vol. 234, 779-815
- **LASKOWSKI et al.** *J. Appl. Cryst.,* 1993, vol. 26, 283-29
- **ROOS et al.** *Parasitol. Today,* 1995, vol. 11, 148-150
- **GNANASEKAR et al.** *Biochem. Biophys. Res. Commun.,* 2009, vol. 386, 333-337
- **CHEIRMARAJ et al.** *J. Trop. Med. Hyg.,* 1992, vol. 95, 47-51
- **VEERAPATHRAN et al.** *PLoS Negl. Trop. Dis.,* 2009, vol. 3, e457
- **CHANDRASEKHAR et al.** *Parasite Immunol.,* 1985, vol. 7, 633-641
- **ABRAHAM et al.** *Immunology,* 1986, vol. 57, 165-169
- **BALAZS et al.** *Protein Eng. Des. Sci.,* 2001, vol. 14, 875-880
- **GNANASEKAR et al.** *Mol. Biochem. Parasit.,* 2008, vol. 159, 98-103
- **OFRAN ; ROST.** *Bioinformatics,* 2007, vol. 23, e13-e16
- **ABRAHAM et al.** *Am. J. Trop. Med. Hyg.,* 1989, vol. 40 (6), 598-604
- **HASLBECK et al.** *Nat. Struct. Mol. Biol.,* 2005, vol. 12, 842-846
- **YOO et al.** *Biotechnol. Lett.,* 2005, vol. 27, 443-448
- **HUANG et al.** *Immunol. Lett.,* 2005, vol. 101, 71-80
- **NORIMINE et al.** *Infect. Immun.,* 2004, vol. 72, 1096-1106
- **SHINNICK et al.** *Infect. Immun.,* 1988, vol. 56, 446-451
- **GNANASEKAR et al.** *Infect. Immun.,* 2004, vol. 72, 4707-15
- **GNANASEKAR et al.** *Mol. Biochem. Parasitol.,* 2008, vol. 159 (2), 98-103

SEQUENCE LISTING

<110> The Board of Trustees of the University of Illinois
Kalyanasundaram, Ramaswamy

<120> Multivalent Vaccine for Filariasis

<130> UIC0030US.NP

<150> US 61/413,681
<151> 2010-11-15

<150> US 61/449,954
<151> 2011-03-07

<150> US 61/522,079
<151> 2011-08-10

<150> PCT/US2011/059501
<151> 2011-11-07

<160> 69

<170> PatentIn version 3.5

<210> 1
<211> 27
<212> DNA
<213> Artificial sequence

<220>
<223> Synthetic oligonucleotide

<400> 1
cgcggatcca tggaagagaa ggtggtg 27

<210> 2
<211> 25
<212> DNA
<213> Artificial sequence

<220>
<223> Synthetic oligonucleotide

<400> 2
ccggaattct cacttgtcgt tggtg 25

<210> 3
<211> 12
<212> PRT
<213> Brugia malayi

<400> 3

```
Trp Ser Ala Glu Gln Trp Asp Trp Pro Leu Gln His
1               5                   10
```

<210> 4
<211> 10
<212> **PRT**
<213> Brugia malayi

<400> 4

```
Glu Val Ile Lys Thr Asn Thr Asn Asp Lys
1               5                   10
```

<210> 5
<211> 8
<212> **PRT**
<213> Brugia malayi

<400> 5

```
Ser Arg Ala Glu His Tyr Gly Glu
1               5
```

<210> 6
<211> 11
<212> **PRT**
<213> Brugia malayi

<400> 6

Lys Leu Pro Ser Asp Val Asp Thr Lys Thr Leu
1 5 10

<210> 7
<211> 9
<212> **PRT**
<213> Brugia malayi

<400> 7

Phe Thr Pro Lys Glu Ile Glu Val Lys
1 5

<210> 8
<211> 8
<212> **PRT**
<213> Brugia malayi

<400> 8

Ile Glu Val Lys Val Ala Gly Asp
1 5

<210> 9
<211> 8
<212> **PRT**
<213> Brugia malayi

<400> 9

Val Gly Leu Asp Ala Ser Phe Phe
1 5

<210> 10
<211> 9
<212> **PRT**
<213> Brugia malayi

<400> 10

Gly Leu Asp Ala Ser Phe Phe Thr Pro
1 5

<210> 11
<211> 9
<212> **PRT**
<213> Brugia malayi

<400> 11

Val Lys Val Ala Gly Asp Asn Leu Val
1 5

<210> 12
<211> 9
<212> **PRT**

<213> Brugia malayi

<400> 12

Lys Leu Pro Ser Asp Val Asp Thr Lys
1 5

<210> 13
<211> 9
<212> PRT
<213> Brugia malayi

<400> 13

Lys Thr Leu Thr Ser Asn Leu Thr Lys
1 5

<210> 14
<211> 9
<212> **PRT**
<213> Brugia malayi

<400> **14**

Val Ile Lys Thr Asn Thr Asn Asp Lys
1 5

<210> 15
<211> 9
<212> **PRT**
<213> Brugia malayi

<400> 15

Asp Thr Lys Thr Leu Thr Ser Asn Leu

1 5

<210> 16
<211> 9
<212> **PRT**
<213> Brugia malayi

<400> 16

Phe Phe Thr Pro Lys Glu Ile Glu Val
1 5

<210> 17
<211> 9
<212> **PRT**
<213> Brugia malayi

<400> 17

Val Gly Leu Asp Ala Ser Phe Phe Thr
1 5

<210> 18
<211> 9
<212> **PRT**
<213> Brugia malayi

<400> 18

Lys Arg Gly His Leu Val Ile Ala Ala
1 5

<210> 19
<211> 9
<212> **PRT**
<213> Brugia malayi

<400> 19

Gly Glu Ile Lys Arg Glu Ile Ser Arg
1 5

<210> 20
<211> 9
<212> PRT
<213> Brugia malayi

<400> 20

Ser Phe Phe Thr Pro Lys Glu Ile Glu
1 5

<210> 21
<211> 9
<212> **PRT**
<213> Brugia malayi

<400> 21

Arg Glu Ile Ser Arg Thr Tyr Lys Leu
1 5

<210> 22
<211> 9
<212> PRT
<213> Brugia malayi

<400> 22

Gly Glu Ile Lys Arg Glu Ile Ser Arg
1 5

<210> 23
<211> 9
<212> **PRT**
<213> Brugia malayi

<400> 23

Ala Glu His Tyr Gly Glu Ile Lys Arg
1 5

<210> 24
<211> 25
<212> DNA
<213> Artificial sequence

<220>
<223> Synthetic oligonucleotide

<400> 24
ccggaattct cacttgtcgt tggtg 25

<210> 25
<211> 26
<212> DNA
<213> Artificial sequence

<220>
<223> Synthetic oligonucleotide

<400> 25
cgcggatcca ccgtgatcca ttgtcg 26

<210> 26
<211> 27
<212> DNA
<213> Artificial sequence

<220>
<223> Synthetic oligonucleotide

<400> 26
aactgcagct gttttccatt tccattc 27

<210> 27
<211> 30
<212> DNA
<213> Artificial sequence

<220>
<223> Synthetic oligonucleotide

<400> 27
aactgcagat gggtaacaag ctcctcatcg 30

<210> 28
<211> 27
<212> DNA
<213> Artificial sequence

<220>
<223> Synthetic oligonucleotide

<400> 28

cgcgaattcg gcgcactgcc aacctgc 27

<210> 29
<211> 24
<212> DNA
<213> Artificial sequence

<220>
<223> Synthetic oligonucleotide

<400> 29
cgcgaattca ccatggtcct ggag 24

<210> 30
<211> 25
<212> DNA
<213> Artificial sequence

<220>
<223> Synthetic oligonucleotide

<400> 30
gctctagatc agtccttctg gctag 25

<210> 31
<211> 26
<212> DNA
<213> Artificial sequence

<220>
<223> Synthetic oligonucleotide

<400> 31
cgggatccat ggaagaaaag gtagtg 26

<210> 32
<211> 26
<212> DNA
<213> Artificial sequence

<220>
<223> Synthetic oligonucleotide

<400> 32
ccctcgagtg ctttcttttt ggcagc 26

<210> 33
<211> 30
<212> DNA
<213> Artificial sequence

<220>
<223> Synthetic oligonucleotide

<400> 33
ccctcgagat gaataaactt ttaatagcat 30

<210> 34

<211> 23
<212> DNA
<213> Artificial sequence

<220>
<223> Synthetic oligonucleotide

<400> 34
gggtacccgc gcattgccaa ccc 23

<210> 35
<211> 29
<212> DNA
<213> Artificial sequence

<220>
<223> Synthetic oligonucleotide

<400> 35
ggggtacccc ggcaaggatc aatttaaaa 29

<210> 36
<211> 28
<212> DNA
<213> Artificial sequence

<220>
<223> Synthetic oligonucleotide

<400> 36
cggaattctc aatctttttg agatgaat 28

<210> 37
<211> 128
<212> PRT
<213> Brugia malayi

<400> 37

```
Met Asn Lys Leu Leu Ile Ala Phe Gly Leu Val Ile Leu Phe Val Thr
1               5                   10                  15

Leu Pro Cys Val Ser Glu Ser Asp Glu Glu Phe Asp Asp Ser Ala Ala
            20                  25                  30

Asp Asp Thr Asp Asp Ser Glu Ala Gly Gly Gly Ser Glu Gly Gly Asp
        35                  40                  45

Glu Tyr Val Thr Lys Gly Glu Phe Val Glu Thr Asp Gly Lys Lys Lys
    50                  55                  60

Glu Cys Ser Ser His Glu Ala Cys Tyr Asp Gln Arg Glu Pro Gln Ala
65                  70                  75                  80

Trp Cys Arg Leu Ser Glu Asn Gln Ala Trp Thr Asp Arg Gly Cys Phe
                85                  90                  95

Cys Glu Asp Lys Leu His Ser Cys Val Ile Glu Arg Thr Asn Asn Gly
            100                 105                 110

Lys Leu Glu Tyr Ser Tyr Cys Ala Pro Glu Ala Gly Trp Gln Cys Ala
        115                 120                 125
```

<210> 38
<211> 572
<212> **DNA**
<213> Brugia malayi

<400> 38

```
ggtttattac cccaagtttg agggaggaga aaatgaataa acttttaata gcattcggtt      60
tggtaattct ttttgtgaca ctcccgtgtg tatcagaatc agacgaagag ttcgatgact     120
ccgcagccga tgacaccgac gacagcgagg ccggaggtgg tagtgaagga ggtgatgaat     180
atgtaaccaa aggggaattt gttgaaactg atggcaaaaa gaaagagtgc tcttcgcacg     240
aagcttgcta cgatcaacgt gaaccacaag cgtggtgcag actgagcgag aatcaggcat     300
ggactgacag aggctgcttc tgcgaagata agttgcattc gtgcgtcatc gaaagaacga     360
acaatggtaa attggagtat tcgtactgtg cacctgaagc aggttggcaa tgcgcatagg     420
aatgaatcag tctttgatac taccaacttt ccatccattt cctacacttt tcaaatttaa     480
ttctttctaa caaataacat ttaactttgg ttttaccgtt attttattaa atattggaaa     540
tatgtgcgac aaaaaaaaaa aaaaaaaaaa aa                                   572
```

<210> 39
<211> 126

<212> **PRT**
<213> Brugia malayi

<400> 39

```
Met Asn Asn Leu Leu Ile Ala Ile Ser Leu Val Ile Leu Leu Val Ile
1               5                   10                  15

Phe Pro Ser Lys Ser Asp Leu Glu Ile Asp Asp Ser Ser Asp Ser Asp
            20                  25                  30

Tyr Ala His Ser Asn Asp Asp Asp Tyr Asn Glu Glu Glu Asp Glu Tyr
        35                  40                  45

Thr Thr Lys Gly Glu Phe Val Glu Thr Asp Gly Lys Trp Lys Asn Cys
    50                  55                  60

Ser Ser His Ala Asp Cys Tyr Asp Tyr Arg Glu Pro Ile Ala Trp Cys
65                  70                  75                  80

Lys Pro Thr Ala Asn Gln Phe Trp Thr Asp Lys Gly Cys Phe Cys Glu
                85                  90                  95

Asp Met Leu Tyr Ser Cys Val Ile Glu Arg Thr Asn Asn Gly Lys Leu
            100                 105                 110

Glu Tyr Ser Tyr Cys Thr Ser Lys Glu Asn Trp Gln Cys Leu
        115                 120                 125
```

<210> 40
<211> 381
<212> DNA
<213> Brugia malayi

<400> 40

```
atgaataatc tattgatagc aatcagttta gtaattcttc ttgtgatatt cccatcaaaa      60
tcagatttag aaattgatga ttcctcagac agtgattatg ctcatagcaa tgacgacgat     120
tacaatgaag aagaagatga atatacgacc aaaggagaat ttgttgaaac tgatggcaaa     180
tggaagaatt gcagttctca tgcagattgc tatgattatc gtgaaccaat agcttggtgc     240
aaacccactg caaatcaatt ttggacagac aaaggttgct tctgtgaaga tatgttgtat     300
tcatgtgtga tcgaaagaac gaacaatggc aaattagaat attcatattg tacatccaaa     360
gaaaactggc aatgtttata a                                               381
```

<210> 41
<211> 128

<212> PRT
<213> Wuchereria bancrofti

<400> 41

```
Met Asn Lys Leu Leu Ile Ala Phe Gly Leu Val Ile Leu Leu Val Thr
1               5                   10                  15

Leu Pro Cys Ala Ser Glu Ser Asp Glu Glu Phe Asp Asp Gly Ser Asn
            20                  25                  30

Asp Glu Thr Asp Asp Lys Glu Asp Glu Gly Asn Ser Glu Gly Gly Asp
        35                  40                  45

Glu Tyr Val Thr Lys Gly Glu Val Val Glu Thr Asp Gly Lys Lys Lys
    50                  55                  60

Glu Cys Ser Ser His Glu Ala Cys Tyr Asp Gln Arg Glu Pro Gln Ala
65                  70                  75                  80

Trp Cys Arg Pro Asn Glu Asn Gln Ser Trp Thr Asp Lys Gly Cys Phe
                85                  90                  95

Cys Glu Asp Lys Leu His Ser Cys Val Ile Glu Arg Lys Asn Asn Gly
            100                 105                 110

Lys Leu Glu Tyr Ser Tyr Cys Ala Pro Glu Ala Gly Trp Gln Cys Ala
        115                 120                 125
```

<210> 42
<211> 387
<212> DNA
<213> Wuchereria bancrofti

<400> 42

```
atgaataaac ttttaatagc attcggttta gtaattcttc ttgtgacact tccgtgtgca      60
tcagaatcag acgaagagtt cgatgacggc tcaaatgatg agaccgacga caaagaggac     120
gaaggtaata gtgaaggagg tgatgaatat gtaaccaaag gagaagttgt tgaaactgat     180
ggcaagaaga aagagtgctc ttcgcacgaa gcttgttacg atcaacgtga accacaagcg     240
tggtgtagac cgaacgagaa tcagtcgtgg actgacaaag gttgcttctg cgaagataag     300
ttgcattcgt gcgtcatcga aagaaagaac aacggtaaat tggagtattc gtattgcgca     360
cctgaagcgg gttggcaatg cgcgtag                                          387
```

<210> 43
<211> 83

<212> PRT
<213> Loa loa

<400> 43

```
Met Ile His Asn Gly Gly Tyr Gln Lys Leu Lys Val Lys Leu Arg Lys
1               5                   10                  15

Lys Glu Cys Lys Ser His Glu Ala Cys Tyr Asp Gln Arg Glu Pro Gln
            20                  25                  30

Asp Trp Cys Arg Leu Asn Glu Asn Gln Ser Trp Thr Asp Lys Gly Cys
        35                  40                  45

Tyr Cys Asp Asp Lys Leu His Ser Cys Ile Ile Glu Arg Lys Asn Gly
    50                  55                  60

Gly Lys Leu Glu Tyr Ala His Cys Ala Pro Glu Gln Gly Trp Lys Cys
65                  70                  75                  80

Pro Ser Lys
```

<210> 44
<211> 252
<212> DNA
<213> Loa loa

<400> 44

```
atgattcata acggtggata ccaaaaatta aaagtcaaat tgaggaagaa agaatgcaag      60
tctcatgaag cttgctacga tcaacgtgaa ccacaagact ggtgcagatt gaacgaaaat     120
caatcgtgga cagacaaagg ttgctactgt gacgataagt tgcattcatg cattattgag     180
cgcaagaacg gtggcaagtt ggaatatgcg cactgtgcgc ctgaacaagg atggaaatgt     240
ccaagcaagt aa                                                         252
```

<210> 45
<211> 229
<212> PRT
<213> Brugia malayi

<400> 45

```
Met Val His Gly Cys Gly Asn Arg Thr Leu Lys Phe Leu Phe Phe Thr
1               5                   10                  15

Ala Asn Leu Leu Thr Cys Ala Phe Gly Ala Leu Leu Phe Gly Ile Ser
            20                  25                  30

Leu Trp Ala Tyr His Asp Glu Asn Phe Leu Gln Lys Leu Glu Lys Val
        35                  40                  45

Gln Glu Ile His Lys Glu Tyr Ser Pro Glu Leu Thr Gln His Gln Ile
    50                  55                  60

Gly Leu Trp Leu Leu Ile Ile Ile Gly Ala Ser Ile Phe Leu Val Gly
65                  70                  75                  80

Phe Leu Gly Cys Cys Gly Ala Ile Cys Glu Ser Thr Lys Leu Leu Ala
                85                  90                  95

Leu Phe Ser Ile Ile Val Leu Ile Leu Ala Ile Leu Glu Val Ala Ser
            100                 105                 110

Ile Val Leu Ile Ile Ala Gly Lys Asp Gln Phe Lys Asn Ala Leu Tyr
        115                 120                 125

Asn Leu Leu Ser Lys Thr Gly Glu Ser Glu Asp Glu Met Gln His Phe
    130                 135                 140

Lys Pro Ile Glu Asp Leu Phe Gln Cys Cys Gly Pro Thr Asn Glu Thr
145                 150                 155                 160

Met Val Arg Tyr Ile Glu Asn Gly Leu Cys Glu Asp Glu Leu Arg Asn
                165                 170                 175

Lys Pro Asn Cys Phe Ala Val Ile Ser Asp His Phe Asp Ser Ser Gln
            180                 185                 190

Lys Asp Ile Ile Lys Ile Ser Val Val Leu Ile Ile Ile Asp Leu Leu
        195                 200                 205

Ala Leu Phe Ser Thr Ser Met Leu Tyr Lys Ala Phe Arg Tyr Gln Thr
    210                 215                 220

Pro Tyr Tyr Tyr Ala
225
```

<210> 46
<211> 690

<212> DNA
<213> Brugia malayi

<400> 46

```
atggttcacg gctgtggtaa tcgaacattg aaatttttat ttttcactgc aaatttatta      60
acttgtgcat ttggtgcact tctttttggc atttcattat gggcttatca tgatgaaaat     120
tttttgcaaa aattagaaaa agtacaggaa atacataaag aatattctcc tgaactaaca     180
caacatcaaa taggattgtg gttattaata attattggtg catccatatt tcttgttggt     240
tttctcggtt gttgtggtgc gatatgtgaa agtaccaagc tattggcttt attttctatt     300
attgtattaa ttcttgcaat acttgaagtt gcttcgatag tacttataat tgctggcaag     360
gatcaattta aaaatgcttt atataattta ctatcaaaaa ctggcgaatc agaagatgaa     420
atgcaacatt ttaaacctat cgaagattta ttccaatgtt gtggtccaac aaatgaaaca     480
atggttcgat acatcgagaa tggcttatgt gaggatgaat taagaaataa accgaattgt     540
ttcgcagtaa tatccgatca ttttgattca tctcaaaaag atatcattaa aatttccgtc     600
gttttaataa ttattgattt gcttgcatta ttttctacat ctatgcttta taaagcattt     660
cgctatcaaa caccatacta ttatgcttaa                                      690
```

<210> 47
<211> 230
<212> PRT
<213> Loa loa

<400> 47

```
Met Val His Gly Cys Gly Asn Arg Met Val Lys Phe Leu Phe Phe Thr
1               5                   10                  15

Ala Asn Leu Leu Ile Cys Leu Phe Gly Ala Leu Ile Phe Gly Phe Ser
            20                  25                  30

Leu Trp Ala Asn Leu Asp Glu Asp Phe Ala Leu Lys Leu Glu Glu Thr
        35                  40                  45

Arg Lys Ile His Lys Glu Asp Phe Asn Val Leu Ala Arg Tyr Gln Ala
    50                  55                  60

Ala Phe Trp Val Leu Val Val Ile Gly Ala Phe Leu Phe Leu Val Gly
65                  70                  75                  80

Phe Leu Gly Cys Cys Gly Ala Met Cys Glu Asn Ile Met Leu Leu Thr
                85                  90                  95

Ala Phe Phe Ile Ile Ile Leu Ile Leu Thr Thr Ile Glu Val Gly Ala
            100                 105                 110

Val Ile Phe Ala Ile Thr Ser Lys Gly Gln Phe Lys Ser Val Leu His
        115                 120                 125

Arg Leu Leu Ser Glu Ala Gly Lys Ala Glu Asp Lys Tyr Leu Pro Asn
    130                 135                 140

Leu Lys Pro Ile Glu Asp Leu Phe Tyr Cys Cys Gly Ala Thr Gln Glu
145                 150                 155                 160

Thr Met Asn Arg Tyr Met Glu His Gly Leu Cys Glu Gly Glu Leu Lys
                165                 170                 175

Asn Lys Pro Asp Cys Phe Thr Ala Ile Ser Asp Tyr Leu Glu Ser Thr
            180                 185                 190

Gly Glu Ala Val Val Val Phe Ala Phe Phe Leu Leu Ile Ile Glu Leu
        195                 200                 205

Phe Ala Leu Val Ser Thr Cys Ile Leu Cys Lys Ala Phe Arg Tyr Gly
    210                 215                 220

Arg Pro Tyr Tyr Tyr Ala
225                 230
```

<210> 48
<211> 693

<212> DNA
<213> Loa loa

<400> 48

```
atggttcacg gttgtggcaa tcgaatggtg aaatttttat ttttcactgc aaatttactg       60
atctgtttat ttggcgcact tatttttggc ttctcattat gggctaattt ggatgaagat      120
tttgcgctaa agctggaaga aacgagaaaa atacataaag aagattttaa tgtgctggca      180
agatatcaag cagcattttg ggtattggtt gttattgggg cattcttgtt tctcgttggt      240
ttcctcggct gttgtggtgc aatgtgtgaa aatatcatgt tgttgactgc atttttcatc      300
ataatactaa ttcttacaac aatcgaagtt ggcgcggtaa tatttgcaat cactagcaag      360
ggccaattta aaagtgtttt acataggcta ctatcagaag ctggtaaagc agaagacaaa      420
tacttgccga atttaaaacc catcgaagat ctgttttatt gctgtggtgc aacgcaagaa      480
acgatgaacc ggtacatgga acatggctta tgtgaaggag aattgaagaa taagccggac      540
tgcttcacgg caatatcgga ttatttggag tcaaccggag aagcggtcgt cgtatttgca      600
ttctttttgc taattatcga gttgtttgct ttagtttcca catgtatact atgtaaagca      660
ttccgttatg gaaggccata ctactatgct taa                                   693
```

<210> 49
<211> 113
<212> PRT
<213> Brugia malayi

<400> 49

```
Met Glu Glu Lys Val Val Glu Leu Thr His Asn Trp Ser Ala Glu Gln
1               5                   10                  15

Trp Asp Trp Pro Leu Gln His Asn Asp Glu Val Ile Lys Val Thr Asn
            20                  25                  30

Thr Asn Asp Lys Phe Glu Val Gly Leu Asp Ala Ser Phe Phe Thr Pro
```

35 40 45

Lys Glu Ile Glu Val Lys Val Ala Gly Asp Asn Leu Val Ile His Cys
50 55 60

Arg His Glu Ser Arg Ala Glu His Tyr Gly Glu Ile Lys Arg Glu Ile
65 70 75 80

Ser Arg Thr Tyr Lys Leu Pro Ser Asp Val Asp Thr Lys Thr Leu Thr
85 90 95

Ser Asn Leu Thr Lys Arg Gly His Leu Val Ile Ala Ala Lys Lys Lys
100 105 110

Ala

<210> 50
<211> 342
<212> DNA
<213> Brugia malayi

<400> 50

atggaagaaa aggtagtgga actaacgcac aactggagtg cggagcagtg ggattggccc 60

ttacagcaca acgatgaggt gattaaagta accaacacga atgataagtt tgaagtcggt 120

ttggatgcat cattcttcac gccgaaggaa atcgaggtaa aagttgctgg cgataatctg 180

gtcattcact gcagacatga atcacgtgcc gagcattatg gagaaattaa acgtgaaatt 240

agtcgtacct ataagcttcc gtcggatgta gatacgaaga ctctgacatc aaatttgacc 300

aagcgaggac atttggtcat cgctgccaaa aagaaagcat ga 342

<210> 51
<211> 165
<212> PRT
<213> Onchocerca volvulus

<400> 51

```
Gln Thr Ser Pro Met Glu Arg Phe Ile Val Asn Leu Leu Asp Ser Thr
1               5                   10                  15

Phe Asp Asp Arg Ser Ser Arg Pro Leu His Ser Val Ala Pro Tyr Trp
            20                  25                  30

Leu His Gln Pro Glu Leu Asn Glu Cys Asn Ile Gly Asn Ser Leu Gly
        35                  40                  45

Glu Val Ile Asn Glu Lys Asp Lys Phe Ala Val Arg Ala Asp Val Ser
    50                  55                  60

His Phe His Pro Lys Glu Leu Ser Val Ser Val Arg Asp Arg Glu Leu
65                  70                  75                  80

Val Ile Glu Gly His His Glu Glu Arg Thr Asp Pro Ala Gly His Gly
                85                  90                  95

Ser Ile Glu Arg His Phe Ile Arg Lys Tyr Val Leu Pro Glu Glu Val
            100                 105                 110

Gln Pro Asp Thr Ile Glu Ser His Leu Ser Asp Lys Gly Val Leu Thr
        115                 120                 125

Ile Ser Ala Asn Lys Thr Ala Ile Gly Thr Thr Ala Ser Arg Asn Ile
    130                 135                 140

Pro Ile Arg Ala Ser Pro Lys Glu Pro Glu Ala Asn Gln Lys Ser Ala
145                 150                 155                 160

Ile Asn Asp Ala Lys
                165
```

<210> 52
<211> 574
<212> DNA
<213> Onchocerca volvulus

<400> 52

caaacttcac cgatggaacg tttcatagtg aaccttcttg acagtacatt cgatgacaga 60

tcatctcgac cgctccattc tgttgcacca tattggcttc atcaaccgga actgaatgaa 120

tgtaatatcg gaaattctct cggcgaggta ataaatgaga aagacaagtt tgcagttcga 180

gctgatgttt ctcatttcca tccgaaggaa ctttcggtgt cggttcgaga tcgtgaacta 240

gttattgaag gtcaccatga agagcgtacc gatccggcag gacatggtag tattgaacgt 300

cacttcattc gaaaatatgt cttgccagaa gaagtgcagc ctgacacgat cgaatcacat 360

ttgtccgata aaggagtgtt gacaattagc gcaaacaaaa cagctattgg cacgactgca 420

tcacgaaaca ttccaattcg agcatctcca aaagaaccgg aagcaaatca gaaatcggca 480

ataaatgatg caaagtaaaa agaagtagaa attccggata tatttcattc tttattttat 540

acgctttttt tttctttttt tgttcatttt aaag 574

<210> 53
<211> 113
<212> PRT
<213> Loa loa

<400> 53

Met Glu Glu Lys Val Leu Glu Leu Thr His Asn Trp Ser Ala Glu Gln
1 5 10 15

Trp Asp Trp Pro Leu Gln His Asn Asp Asp Val Ile Lys Val Thr Asn
20 25 30

Thr Asn Asp Lys Phe Glu Val Ser Leu Asp Ala Ser Phe Phe Thr Pro
35 40 45

Lys Glu Ile Glu Val Lys Val Ala Gly Asp Asn Leu Val Ile His Cys
50 55 60

Arg His Glu Ser Arg Ala Asp Gln Tyr Gly Glu Ile Lys Arg Glu Ile
65 70 75 80

Ser Arg Thr Tyr Lys Leu Pro Ser Asp Ile Asp Thr Lys Thr Leu Thr
85 90 95

Ser Thr Leu Thr Lys Arg Gly His Leu Val Ile Val Ala Lys Lys Lys
100 105 110

Ala

<210> 54
<211> 342
<212> DNA

<213> Loa loa

<400> 54

```
atggaagaaa aggtattgga actaacgcat aattggagtg ccgagcagtg ggattggccc      60
ttgcagcata acgatgacgt gattaaagtg accaatacga atgataagtt tgaagtcagt     120
ctggatgctt cattcttcac accgaaggaa atcgaggtga aagttgctgg tgataatctg     180
gtcattcact gcagacatga atcacgtgcc gaccaatacg gtgaaattaa acgtgaaatt     240
agtcgtacct ataagcttcc gtcggatatt gatacgaaga ccctgacatc aactttgacc     300
aaaagaggac atctggtcat cgttgccaaa aagaaggcat ga                        342
```

<210> 55
<211> 220
<212> PRT
<213> Brugia malayi

<400> 55

```
Met Leu Phe Phe Val Ile Phe Ser Ile Thr Ile Ala Val Val Ala Gly
1               5                   10                  15
```

```
Phe Glu Cys Pro Gly Gly Arg Leu Thr Pro Gln Gln Arg Lys Asp Ile
            20                  25                  30

Val Arg Gln Asn Asn Lys Phe Arg Ser Leu Leu Ile His Gly Lys Leu
        35                  40                  45

Lys Asn Arg Asn Gly Thr Tyr Met Pro Arg Gly Lys Asn Met Leu Leu
    50                  55                  60

Leu Lys Trp Ser Cys Gln Leu Glu Asn Ser Ala Gln Arg Trp Ala Asn
65                  70                  75                  80

Gln Cys Val Phe Gly His Ser Pro Arg Asn Gln Arg Gln Gly Ile Gly
                85                  90                  95

Glu Asn Val Tyr Ala Tyr Trp Ser Ser Glu Ser Val Glu Lys Leu Arg
            100                 105                 110

Asn Thr Ala Gly Thr Glu Ala Gly Lys Ser Trp Trp Ser Glu Leu Pro
        115                 120                 125

Lys Leu Tyr Lys Gln Asn Pro Ser Asn Asn Leu Thr Asp Asp Val Ala
    130                 135                 140

Arg Gln Gly Val Leu His Phe Thr Gln Met Ala Trp Gly Lys Thr His
145                 150                 155                 160

Lys Ile Gly Cys Gly Ile Ala Thr Asn Cys Asp Gly Gly Arg Thr Leu
                165                 170                 175

Ile Ala Ile Cys His Tyr Ser Pro Ala Gly Asn Met Leu Lys Glu Leu
            180                 185                 190

Ile Tyr Glu Leu Gly Glu Pro Cys Lys Thr Asp Ser Asp Cys Asn Thr
        195                 200                 205

Lys Lys Cys Ala Lys Lys Ser Gly Leu Cys Arg Lys
    210                 215                 220
```

<210> 56
<211> 897
<212> DNA
<213> Brugia malayi

<400> 56

```
gcacgagata ttttcaaagc taacagtata cattccacca aaaaactcgg caaaatgtta        60
tttttcgtta tattttccat cacgattgct gttgtggcag gttttgaatg tccaggaggt       120
cgactaacac cacagcaacg taaagatatc gttcgtcaga acaataaatt tcgttcatta       180
ctaattcatg gaaaacttaa aaataggaat ggtacatata tgccacgcgg caagaatatg       240
ttgctactga agtggagttg tcagttagaa aattctgcgc aaagatgggc aaatcaatgc       300
gtctttgggc actctccgag aaatcaaaga caaggaatcg gtgaaaatgt ctatgcctac       360
tggtcatcag aaagcgtcga aaagcttaga aataccgctg gtacggaagc cggcaaaagt       420
tggtggtcag aacttccgaa actgtacaaa cagaatcctt cgaacaatct gactgatgat       480
gttgccagac aaggtgtttt acatttcaca cagatggcct ggggtaaaac gcataaaatt       540
ggttgtggta tagcaacaaa ctgcgatggt ggtcgcacac tcatagctat ttgccattat       600
tcacccgctg gaaatatgct aaaagaactg atatatgagc ttggcgaacc atgcaaaacg       660
gatagcgatt gtaacacaaa gaaatgtgcc aaaaaatctg gattgtgtag aaaatgaaag       720
agagtttaaa gtttcacttt ttgcactctt aattttggtt ttatattata aaactgagaa       780
ttattacaaa catatttatt ggctattata agataaaaaa gttccttgaa attcttctca       840
aataaaattt ctctctgata aaagcatgaa acaaaaaaaa aaaaaaaaaa aaaaaaa          897
```

<210> 57
<211> 220
<212> PRT
<213> Wuchereria bancrofti

<400> 57

```
Met Leu Leu Phe Val Ile Phe Ser Ala Thr Ile Val Ala Val Ala Ala
1               5                   10                  15

Phe Glu Cys Pro Gly Gly Gln Leu Thr Pro Gln Gln Arg Lys Asp Ile
            20                  25                  30

Val Arg Gln Asn Asn Lys Phe Arg Ser Leu Leu Ile Arg Gly Lys Leu
        35                  40                  45

Lys Asn Arg Asn Gly Thr Tyr Met Pro Arg Gly Lys Asn Met Leu Gln
    50                  55                  60

Leu Thr Trp Ser Cys Gln Leu Glu Asn Ser Ala Gln Arg Trp Ala Asn
65                  70                  75                  80

Gln Cys Val Phe Gly His Ser Pro Arg Asn Gln Arg Gln Gly Ile Gly
                85                  90                  95

Glu Asn Val Tyr Ala Tyr Trp Ser Ser Ala Ser Val Glu Asn Leu Arg
            100                 105                 110

Lys Thr Ala Gly Thr Glu Ala Gly Lys Ser Trp Trp Ser Glu Leu Pro
        115                 120                 125

Glu Leu Tyr Lys His Asn Pro Ser Asn Asn Leu Thr Asp Asp Val Ser
    130                 135                 140

Arg Gln Gly Val Leu His Phe Thr Gln Met Ala Trp Gly Lys Thr His
145                 150                 155                 160

Lys Ile Gly Cys Gly Ile Ala Thr Asn Cys Asp Gly Gly Arg Thr Leu
                165                 170                 175

Ile Thr Ile Cys His Tyr Ser Pro Ala Gly Asn Ile Leu Lys Asn Leu
            180                 185                 190

Ile Tyr Glu Leu Gly Glu Pro Cys Lys Lys Asp Gly Asp Cys Asn Thr
        195                 200                 205

Lys Lys Cys Ala Lys Lys Ser Gly Leu Cys Arg Lys
    210                 215                 220
```

<210> 58
<211> 873
<212> DNA
<213> Wuchereria bancrofti

<400> 58

```
ggaattcggc acgagcgaca tcttctgtta tcaaatatat tttcaaagct aacagtgtac     60
attctgccag aaaatcccgc aaaatgttac ttttcgtcat attttctgca actattgttg    120
ctgtggcggc ttttgaatgt ccaggaggtc aactaacacc gcaacaacgt aaagatatcg    180
ttcgtcagaa caataaattt cgttcattac tgattcgtgg aaaacttaaa aataggaatg    240
gtacatatat gccacgcggc aagaatatgt tgcaactgac atggagttgt cagttagaaa    300
attctgcgca aagatgggca aatcagtgcg tctttggaca ctcaccgaga aatcaaagac    360
aaggaatcgg tgaaaatgtt tacgcttact ggtcgtcagc aagcgttgaa aatcttagaa    420
aaaccgctgg tacggaagcc ggcaaaagtt ggtggtcaga acttccggaa ctgtacaaac    480
acaatccttc gaacaatctg actgacgatg tttccaggca aggtgttttg catttcacac    540
agatggcttg ggtaaaacg cataaaattg gttgtggtat tgctacaaac tgtgatggtg     600
gtcgcacact cataactatt tgccattatt cgcccgctgg aaatatactg aaaaatctga    660
tatatgagct tggcgaaccg tgcaaaaagg atggcgattg taacacaaag aaatgtgcga    720
aaaaatctgg attgtgcaga aagtgaaaga atttgaagag tttcaccttc tcatttctaa    780
ttttggtttt gtatcataaa actgacaatt attacaaaca tatttattgg ctattatggg    840
ataaagaagt ttcttgaaaa aaaaaaaaaa aaa                                 873
```

<210> 59
<211> 220
<212> PRT
<213> Onchocerca volvulus

<400> 59

```
Met Ile Leu Phe Ile Ile Phe Pro Ala Ile Val Val Ala Val Thr Gly
1               5                   10                  15

Tyr Asn Cys Pro Gly Gly Lys Leu Thr Ala Leu Glu Arg Lys Lys Ile
            20                  25                  30

Val Gly Gln Asn Asn Lys Tyr Arg Ser Asp Leu Ile Asn Gly Lys Leu
        35                  40                  45

Lys Asn Arg Asn Gly Thr Tyr Met Pro Arg Gly Lys Asn Met Leu Glu
    50                  55                  60

Leu Thr Trp Asp Cys Lys Leu Glu Ser Ser Ala Gln Arg Trp Ala Asn
65                  70                  75                  80

Gln Cys Ile Phe Gly His Ser Pro Arg Gln Gln Arg Glu Gly Val Gly
                85                  90                  95

Glu Asn Val Tyr Ala Tyr Trp Ser Ser Val Ser Val Glu Gly Leu Lys
            100                 105                 110

Lys Thr Ala Gly Thr Asp Ala Gly Lys Ser Trp Trp Ser Lys Leu Pro
        115                 120                 125

Lys Leu Tyr Glu Asn Asn Pro Ser Asn Asn Met Thr Trp Lys Val Ala
    130                 135                 140

Gly Gln Gly Val Leu His Phe Thr Gln Met Ala Trp Gly Lys Thr Tyr
145                 150                 155                 160

Lys Ile Gly Cys Gly Val Ala Thr Gln Cys Asp Gly Gly Arg Thr Leu
                165                 170                 175

Ile Val Ile Cys His Tyr Ser Pro Gly Gly Asn Met Val Gly Glu Val
            180                 185                 190

Ile Tyr His Arg Gly Asn Pro Cys Lys Val Asp Lys Asp Cys Tyr Thr
        195                 200                 205

Lys Lys Cys Leu Ser Lys Ser Gly Leu Cys Arg Lys
    210                 215                 220
```

<210> 60
<211> 883
<212> DNA
<213> Onchocerca volvulus

<400> 60

```
gcattttcta aagtgacatc ttctgttatt atcaaatata tttctgagat taacagctta        60
tattctatcg gaaaattccg caaaatgata cttttcatca tcttccctgc aattgtcgtc       120
gcggtgacgg gttataattg tccaggaggt aaactaacag cactggaacg taaaaaaatc       180
gttggtcaga ataataaata tcgttcggat ttgattaatg gaaagcttaa gaatagaaat       240
ggtacataca tgccaagagg caagaatatg ttggaactga catgggattg taaattggaa       300
agttcggcac aaaggtgggc caatcagtgt atctttggac attcaccaag acaacaaaga       360
gagggagttg gtgaaaatgt ttatgcgtac tggtcatcag tatcagttga aggtcttaaa       420
aaaactgctg gtacggatgc tgggaaaagc tggtggtcaa agcttccaaa attatatgaa       480
aataaccctt cgaataatat gacttggaaa gttgccggtc aaggtgtttt gcatttcaca       540
caaatggctt ggggtaagac ttataaaatt ggttgcggtg ttgcaacaca atgtgatggt       600
ggtagaacac ttattgttat ttgtcactat tctcctggtg gaaatatggt tggagaggtg       660
atataccacc gaggtaatcc gtgtaaagtc gacaaagatt gctatacgaa aaaatgttta       720
tcaaaatctg gactgtgcag aaaatgaaaa tttttcgctt ttcttcattt aattctcggc       780
tatatatctc ctatattaat ttttcagcaa aaaagctata aagaaatatt cataattaaa       840
taaatatagt aattattatg aaataaaaaa ttaagttctg ctc                         883
```

<210> 61
<211> 220
<212> PRT
<213> Loa loa

<400> 61

```
Met Leu Cys Ser Phe Met Phe Ala Ala Ile Ile Ile Ala Val Glu Gly
1               5                   10                  15

Tyr Arg Cys Gln Gly Gly Arg Leu Thr Pro Glu Gln Arg Lys Ala Ile
            20                  25                  30

Val Ile Gln Asn Asn Lys Phe Arg Ser Gln Leu Ile Arg Gly Glu Leu
        35                  40                  45

Lys Asn Lys Ala Gly Glu Phe Met Pro Arg Gly Lys Asn Met Leu Arg
    50                  55                  60

Met Arg Trp Ser Cys Ser Leu Glu Tyr Ser Ala Gln Arg Trp Ala Asp
65                  70                  75                  80
```

```
Arg Cys Ile Phe Gly His Ser Pro Arg Asp Gln Arg Asn Asn Ile Gly
                85                  90                  95

Glu Asn Val Tyr Ala Tyr Trp Ser Ser Gly Ser Val Glu Gly His Arg
            100                 105                 110

Lys Thr Ala Gly Thr Asp Ala Gly Lys Asn Trp Trp Ser Glu Leu Pro
        115                 120                 125

Glu Arg Tyr Gly Ser Asn Pro Ser Asn Asn Leu Thr Ala Gln Val Ser
    130                 135                 140

Ser Gln Gly Val Leu His Phe Thr Gln Met Ala Trp Gly Lys Thr Tyr
145                 150                 155                 160

Lys Ile Gly Cys Gly Ile Ala Thr Asn Cys Asp Gly Gly Arg Thr Leu
                165                 170                 175

Met Val Ile Cys His Tyr Ser Pro Ala Gly Asn Met Leu Lys Glu Leu
            180                 185                 190

Ile Tyr Glu Leu Gly Glu Pro Cys Lys Lys Asn Asn Asp Cys Tyr Thr
        195                 200                 205

Lys Lys Cys Ser Val Lys Ser Gly Leu Cys Asn Lys
    210                 215                 220
```

<210> 62
<211> 663
<212> **DNA**
<213> Loa loa

<400> 62

```
atgttatgtt ccttcatgtt tgcagcaatt atcattgctg tggaaggtta tcgatgtcaa      60
ggaggtcgac taacaccgga gcaacgtaaa gcaatcgtta ttcaaaataa taaattccgt     120
tcgcaactga tccgtggcga gcttaagaac aaagctggtg aatttatgcc acgtggcaag     180
aatatgttga gaatgagatg gagttgttcg ctggaatatt ccgcgcagag atgggcagat     240
agatgtatct ttggacactc accaagagat caaagaaaca atatcggtga aaatgtctat     300
gcttactggt catcaggaag cgttgaaggt catagaaaaa ctgctggtac agatgctggt     360
aaaaattggt ggtcagaact tccggaacgg tacggatcta atccttcaaa taatttgact     420
gctcaagttt ccagccaagg tgttttgcat ttcacacaga tggcttgggg taaaacgtac     480
aaaattggct gtggtattgc tacaaactgc gatggtggca ggacactaat ggtcatttgc     540
cattattcgc cagctgggaa tatgttgaaa gagcttatat atgagcttgg cgaaccatgc     600
```

aagaagaaca atgattgtta taccaagaag tgttcagtca agtctggatt gtgcaacaag 660

tga 663

<210> 63
<211> 89
<212> PRT
<213> Wuchereria bancrofti

<400> 63

```
Met Ile Leu Glu Val Ala Ser Ile Val Leu Ile Ile Ala Gly Lys Asp
1               5                   10                  15

Gln Phe Lys Asn Ala Leu Tyr Asn Leu Leu Ser Lys Thr Gly Glu Ser
            20                  25                  30

Glu Asp Glu Met Gln His Phe Lys Pro Ile Glu Asp Leu Phe Gln Cys
        35                  40                  45

Cys Gly Pro Thr Asn Glu Thr Met Val Arg Tyr Ile Glu Asn Gly Leu
    50                  55                  60

Cys Glu Asp Glu Leu Arg Asn Lys Pro Asn Cys Phe Ala Val Ile Ser
65                  70                  75                  80

Asp His Phe Asp Ser Ser Gln Lys Asp
                85
```

<210> 64
<211> 53
<212> PRT
<213> Wuchereria bancrofti

<400> 64

```
Val Ile His Cys Arg His Glu Ser Arg Ala Glu His Tyr Gly Glu Ile
1               5                   10                  15

Lys Arg Glu Ile Ser Arg Thr Tyr Lys Leu Pro Ser Asp Val Asp Thr
            20                  25                  30

Lys Thr Leu Thr Ser Asn Leu Thr Lys Arg Gly His Leu Val Ile Ala
        35                  40                  45

Ala Lys Lys Lys Ala
    50
```

<210> 65
<211> 267

<212> **DNA**
<213> Wuchereria bancrofti

<400> 65

```
atgatcctgg aggtggcctc catcgtgctg atcatcgccg gcaaggacca gttcaagaac        60
gctctgtaca acctgctgtc taagacaggc gagagcgagg acgagatgca gcacttcaag       120
cctatcgagg acctgttcca gtgctgcggc cctaccaacg agaccatggt gaggtacatc       180
gagaacggac tgtgcgagga cgagctgagg aacaagccta actgtttcgc cgtgatctct       240
gaccacttcg attctagcca gaaggac                                           267
```

<210> 66
<211> 162
<212> **DNA**
<213> Wuchereria bancrofti

<400> 66

```
gtcattcact gcagacatga atcacgtgcc gagcattatg gagaaattaa acgtgaaatt        60
agtcgtacct ataagcttcc gtcggatgta gatacgaaga ctctgacatc aaatttgacc       120
aagcgaggac atttggtcat cgctgccaaa aagaaagcat ga                          162
```

<210> 67
<211> 387
<212> DNA
<213> Brugia malayi

<400> 67

```
atgggtaaca agctcctcat cgctttcgga ctcgtgatcc tgtttgtcac cctgccttgc        60
gtcagtgaga gcgacgagga gtttgatgac tctgccgctg acgacaccga cgattccgag       120
gccggtggcg gctctgaggg gggcgacgag tacgtgacta agggggagtt tgtggagacc       180
gatggaaaga agaaagagtg ctccagccac gaggcctgtt atgaccagcg ggagcctcag       240
gcttggtgca ggctgtctga gaatcaggcc tggactgata gaggctgttt ctgtgaggac       300
aagctgcatt cttgcgtgat tgagcggact aataatggca agctggaata cagctactgc       360
gctcccgagg caggttggca gtgcgcc                                           387
```

<210> 68
<211> 153
<212> DNA
<213> Wuchereria bancrofti

<400> 68

```
atggtcattc actgcaggca cgagagcaga gccgagcact acggcgagat caaaagagag       60
atcagcagga catacaagct gccctcagac gtggacacta agactctgac tagcaatctg      120
accaagaggg gccacctggt catcgctgcc aag                                   153
```

<210> 69
<211> 267
<212> **DNA**
<213> Wuchereria bancrofti

<400> 69

```
atgatcctgg aggtggcctc catcgtgctg atcatcgccg gcaaggacca gttcaagaac       60
gctctgtaca acctgctgtc taagacaggc gagagcgagg acgagatgca gcacttcaag      120
cctatcgagg acctgttcca gtgctgcggc cctaccaacg agaccatggt gaggtacatc      180
gagaacggac tgtgcgagga cgagctgagg aacaagccta actgtttcgc cgtgatctct      240
gaccacttcg attctagcca gaaggac                                          267
```